# EUROPEAN PATENT APPLICATION

(11) **EP 2 087 833 A2**
(43) Date of publication of application: **12.08.2009**
(21) Application number: 09001421.8
(22) Date of filing: 02.02.2009
(51) Int. Cl.: A61B 1/04, G05B 15/02

(54) **Medical support control system**

(30) Priority: 05.02.2008 US 26068; 19.02.2008 US 33083; 05.02.2008 US 25922
(71) Applicant: Olympus Medical Systems Corporation, Tokyo 151-0072 (JP)
(72) Inventor: Sekiguchi, Kiyoshi, Tokyo 151-0072 (JP); Tashiro, Koichi, Tokyo 151-0072 (JP); Ito, Masaru, Tokyo 151-0072 (JP); Yamaki, Masahide, Tokyo 151-0072 (JP); Furukawa, Nobuyuki, Tokyo 151-0072 (JP)
(74) Representative: von Hellfeld, Axel

(57) **Abstract**

A medical support control system, comprising: a first controller (114) connected to at least one device; a second controller (202) connected to at least one device; and a manipulation display device (221) shared by the first and second controllers, wherein: the manipulation display device is caused to alternately display a first graphical user interface created by the first controller and a second graphical user interface created by the second controller.

## Description

### Background of the Invention

### Field of the Invention

The present invention relates to a medical support control system for controlling medical devices and non-medical devices used for operations.

### Description of the Related Art

Operating systems using medical controllers or the like for controlling medical devices such as endoscopes or the like used for operations have been proposed. Medical devices to be controlled such as electric knives, insufflation devices, endoscope cameras, light source devices, or the like are connected to the medical controller (also referred to as MC). Also, a display device, a manipulation panel, or the like is connected to the MC. The manipulation panel includes a display unit and a touch sensor, and is used as a central manipulation device by nurses or the like working in an unsterilized area. The display device is used for displaying endoscope images or the like.

There is audio-visual equipment in the operating room such as a room light, a room camera, an interphone device, a liquid crystal display device, or the like (non-medical devices). The audio-visual equipment is controlled independently or by a non-medical controller (also referred to as an NMC) used for the central control.

Japanese Patent Application Publication No. 2006-000536, for example, discloses an operating system, comprising:
a first controller connected to a medical device provided in an operating room;
a second controller connected to a non-medical device provided in the operating room; and
manipulation instruction input means transmitting the content of a manipulation instruction for the medical device or the non-medical device to the first controller when a manipulation instruction is input. The first controller transmits to the second controller a first control signal in accordance with the manipulation instruction of the non-medical device input into the manipulation instruction means. The second controller converts the first control signal into a second control signal used for controlling the non-medical device, and transmits the second control signal to the non-medical device. Thereby, the operating system and a non-medical system work together, and the operating person himself/herself or the like can manipulate the non-medical devices.

### Summary of the Invention

One aspect of the present invention is a medical support control system, comprising:
a first controller connected to at least one device;
a second controller connected to at least one device; and
a manipulation display device shared by the first and second controllers, wherein:
   the manipulation display device is caused to alternately display a first graphical user interface created by the first controller and a second graphical user interface created by the second controller.

The first controller has first identification information and user environment information corresponding to the first identification information;
the second controller has second identification information and user environment information corresponding to the second identification information; and
when log-in to at least one of the first and second controllers has succeeded, an operation environment for the controller to which the log-in has succeeded is set in accordance with the user environment information.

An operation environment is set for controllers other than the controller to which the log-in has succeeded, in accordance with user environment information common to all users.

The identification information includes at least an operating person name and a procedure name; and
the user environment information is operation environment information of the controller to which the log-in has succeeded, used for setting an operation environment appropriate for a procedure performed by an operating person identified by the operating person name and the procedure name.

One aspect of the present invention is a medical support control system, comprising:
a first controller connected to at least one device;
a second controller connected to at least one device, connected to a network, and provided in a medical environment different from that of the first controller; and
a manipulation display device shared by the first and second controllers and alternately displaying a first graphical user interface (first GUI) created by the first controller and a second graphical user interface (second GUI) created by the second controller, wherein:
   if a prescribed report is received via the network or from the prescribed connected device while the first GUI is being displayed on the manipulation display device, a symbol indicating that the report has been received is displayed on the first GUI, and when the symbol is pressed, the first GUI is switched to a prescribed window of the second GUI corresponding to the report, and a process corresponding to the report is performed by the second controller.

### Brief Description of the Drawings

Fig. 1 shows an entire configuration of a medical device control system according to the embodiment of present invention;
Fig. 2 is a block diagram showing an entire configuration of a medical support system 100 according to the embodiment of present invention;
Fig. 3 is a block diagram showing an NMC 202 in the first embodiment;
Fig. 4 shows switching of a control target by a TP control switching unit 305 performed between the NMC 202 and an MC 114 in the first embodiment;
Fig. 5 shows examples of windows displayed on a TP 221 in the first embodiment;
Fig. 6 shows a flowchart for log-in and windows displayed on the TP 221 in the first embodiment;
Fig. 7 is a flowchart for setting an operation environment in the first embodiment; and
Fig. 8 shows the data structure of an MC-side database and an NMC side database in the first embodiment.
Fig. 9 is a block diagram showing a configuration of a NMC 202 according to the second embodiment;
Fig. 10 shows an example of a setting environment for an MC 114 and the NMC 202 according to the second embodiment;
Fig. 11 shows switching operation of the control target of a TP control switching unit 305 performed between the NMC 202 and the MC 114 according to the second embodiment;
Fig. 12 shows an example of a display window (first GUI) of a touch panel (TP_{MC}) when the MC 114 side is the control target according to the second embodiment;
Fig. 13 shows an example of a telephone conversation window (second GUI) of the touch panel (TP_{NMC}) displayed when the NMC 202 side is the control target according to the second embodiment;
Fig. 14 is a flowchart for switching of the control target performed by the TP control switching unit 305 between the MC 202 and the MC 114 according to the second embodiment;
Fig. 15 shows a switching operation from a window of the MC 114 side to a window of the NMC 202 side performed when e-mails are received while the window of the MC 114 side is being displayed according to the second embodiment (variation 1);
Fig. 16 is a flowchart for switching of the control target between the NMC 202 and the MC 114 performed by the TP control switching unit 305 according to the second embodiment (variation 1);
Fig. 17 shows switching to a window of the NMC 202 side performed when there is no more free space in a storage medium while a window of the MC 114 side is being displayed according to the second embodiment (variation 2); and
Fig. 18 is a flowchart for switching of a control target between the NMC 202 and the MC 114 performed by the TP control switching unit 305 according to the second embodiment (variation 2).
Fig. 19A is a first view showing switching of a control target performed by a TP control switching unit 305 between the NMC 202 and the MC 114 according to the third embodiment;
Fig. 19B is a second view showing switching of a control target performed by the TP control switching unit 305 between the NMC 202 and the MC 114 according to the third embodiment;
Fig. 20 shows an example of an error display displayed on an operation window according to the third embodiment;
Fig. 21 shows an example of the data structure of error information Eg according to the third embodiment;
Fig. 22 shows error levels according to the third embodiment;
Fig. 23 shows differences of register positions according to error levels when error information is registered on error information output table Tb1 according to the third embodiment;
Fig. 24 is a flowchart for outputting error information according to the third embodiment; and
Fig. 25 shows, in detail, process A (S207) in Fig. 24.

### Description of the Preferred Embodiments

Hereinafter, the embodiments of the present invention will be explained in detail, referring to the drawings.

### <The first embodiment>

In the present embodiment, explanations are given to a medical support control system in which a plurality of controllers constituting the medical support control system are operated and log-in to one of the controllers allows log-in to another controller.

A medical support control system according to the present embodiment includes a medical device control system and a non-medical device control system. The medical device control system includes a plurality of medical devices and a medical controller for controlling these medical devices. The non-medical device control system includes non-medical devices (that may further include medical devices) that are used for operations, and a non-medical controller for controlling these non-medical devices.

An endoscopic operating system will be explained as an example of the medical device control system.

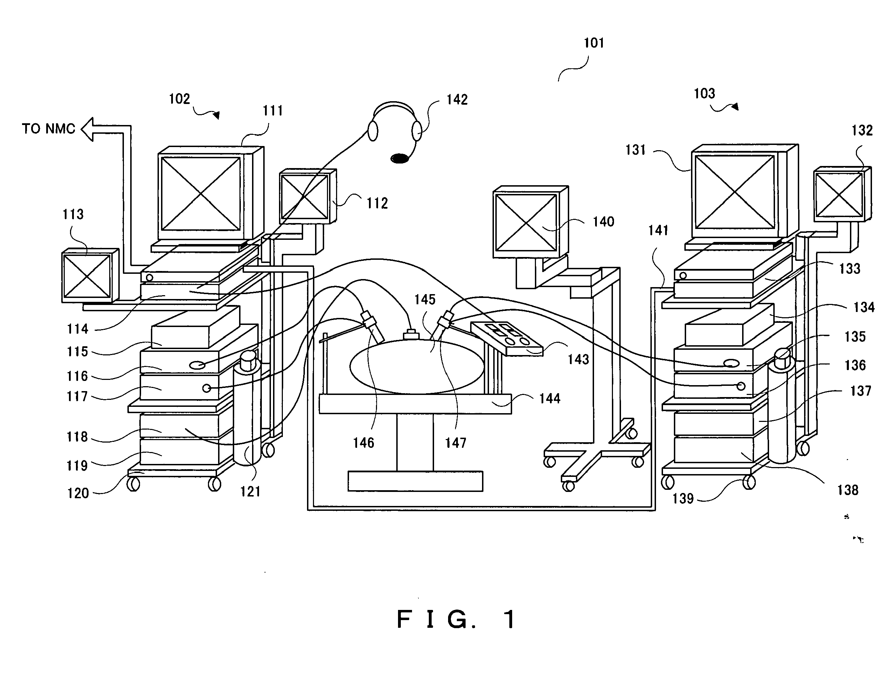

Fig. 1 shows an entire configuration of the medical device control systemaccording to the present embodiment. An endoscopic operating system is shown as a medical device control system 101. In the operating room, a first endoscopic operating system 102 and a second endoscopic operating system 103 beside a bed 144 on which a patient 145 is laid and a wireless remote controller 143 for the operating person are provided.

The endoscopic operating systems 102 and 103 respectively have first and second trolleys 120 and 139 each including a plurality of endoscope peripheral devices used for observation, examination, procedures, recoding, and the like. Also, an endoscope image display panel 140 is arranged on a movable stand.

On the first trolley 120, an endoscope image display panel 111, a central display panel 112, a central manipulation panel device 113, a medical controller (MC) 114, a recorder 115, a video processor 116, an endoscope light source device 117, an insufflation unit 118, and an electrical surgical device 119 are arranged.

The central manipulation panel device 113 is arranged in an unsterilized area to be used by nurses or the like in order to manipulate the respective medical devices in a centralized manner. This central manipulation panel device 113 may include a pointing device such as a mouse, a touch panel, or the like (not shown). By using the central manipulation panel device 113, the medical devices can be managed, controlled, and manipulated in a centralized manner.

The respective medical devices are connected to the MC 114 via communication cables (not shown) such as serial interface cables or the like, and can have communications with one another.

Also, a headset-type microphone 142 can be connected to the MC 114. The MC 114 can recognize voices input through the headset-type microphone 142, and can control the respective devices in accordance with the voices of the operating person.

The endoscope light source device 117 is connected to a first endoscope 146 through a light-guide cable used for transmitting the illumination light. The illumination light emitted from the endoscope light source device 117 is provided to the light guide of the first endoscope 146 and illuminates the affected areas or the like in the abdomen of the patient 145 into which the insertion unit of the first endoscope 146 has been inserted.

The optical image data obtained through the camera head of the first endoscope 146 is transmitted to a video processor 116 through a camera cable. The optical image data undergoes signal processing in a signal processing circuit in the video processor 116, and the video signals are created.

The insufflation unit 118 provides CO₂ gas to the abdomen of the patient 145 through a tube. The CO₂ gas is obtained from a gas tank 121.

On the second trolley 139, an endoscope image display panel 131, a central display panel 132, a expansion unit 133, a recorder 134, a video processor 135, an endoscope light source device 136, and other medical devices 137 and 138 (such as an ultrasonic processing device, a lithotripsy device, a pump, a shaver, and the like) are arranged. These respective devices are connected to the expansion unit 133 through cables (not shown), and can communicate with one another. The MC 114 and the expansion unit 133 are connected to each other through the expansion cable 141.

The endoscope light source device 136 is connected to a second endoscope 147 through the light-guide cable for transmitting the illumination light. The illumination light emitted from the endoscope light source device 136 is provided to the light guide of the second endoscope 147, and illuminates the affected areas or the like in the abdomen of the patient 145 into which the insertion unit of the second endoscope 147 has been inserted.

The optical image data obtained through the camera head of the second endoscope 147 is transmitted to a video processor 135 through a camera cable. The optical image data undergoes signal processing in a signal processing circuit in the video processor 135, and the video signals are created. Then, the video signals are output to the endoscope image display panel 131, and endoscope images of the affected areas or the like are displayed on the endoscope image display panel 131.

Further, the MC 114 can be controlled by the operating person manipulating the devices in the unsterilized area. Also, the first and second trolleys 120 and 139 can include other devices such as printers, ultrasonic observation devices, or the like.

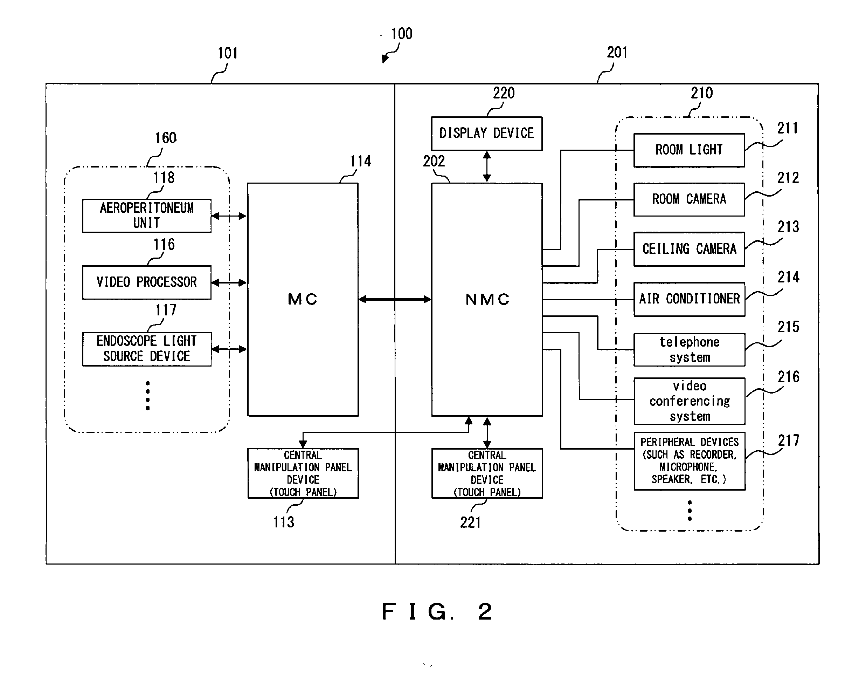

Fig. 2 is a block diagram showing an entire configuration of a medical support control system 100 according to the present embodiment. As described above, the medical support control system 100 includes the medical device control system 101 and a non-medical device control system 201. A detailed configuration of the medical device control system 101 is as shown in Fig. 1. However, in Fig. 2, the medical device control system 101 is shown in a simplified manner for simplicity of explanation.

In Fig. 2, a medical device group 160 is a group of medical devices that are directly connected to the medical controller 114 or are indirectly connected to the MC 114 via the expansion unit 133. Examples of the devices included in the medical device group 160 are the insufflation unit 118, the video processor 116, the endoscope light source device 117, the electrical surgical device 119, and the like.

The central manipulation panel device 113 has a touch panel, and in accordance with the information input into the touch panel, the devices connected to the MC 114 or a non-medical device controller (NMC) 202 that will be described later can be manipulated.

The non-medical control system 201 includes the NMC 202 connected to the MC 114 through a communication cable or the like, and a non-medical device group 210. In this configuration, the NMC 202 can transmit and receive, through an image cable, the video signals to and from the medical device group 160 connected to the MC 114.

The NMC 202 controls the non-medical devices (including the audio-visual devices) connected thereto. As shown in Fig. 2, the non-medical device group 210 connected to the NMC 202 according to the present embodiment consists of a room light 211, a room camera 212, a ceiling camera 213, an air conditioner 214, a telephone system 215, a conference system 216 to be used for individuals in remote places (referred to as a video conference system hereinafter), and other peripheral devices 217. Further, a display device 220 and a central manipulation panel device 221 are connected to the NMC 202.

Also, the non-medical device group 210 includes equipment such as light devices provided in the operating room in addition to the AV devices used for recording and reproducing image data.

The display device 220 is a plasma display panel (PDP) or a liquid crystal display (LCD) device, and displays images of the predetermined device or images of the devices selected by nurses or the like through the central manipulation panel device 221. The room light 211 is a device that illuminates the operating room. The room camera 212 is used for shooting images of the situations in the operating room. The ceiling camera 213 is a camera suspended from the ceiling whose positions can be changed. The conference system 216 is a system that displays images and transmits voices of nurses or the like in the medical office or the nurse stations, and enables conversations with them. The peripheral devices 217 are, for example, a printer, a CD player, a DVD recorder, and the like. The central manipulation panel device 221 has a touch panel that is the same as that included in the central manipulation panel device 113, and controls the respective AV devices connected to the NMC 202. The central manipulation panel devices 113 and 221 are referred to as TPs hereinafter.

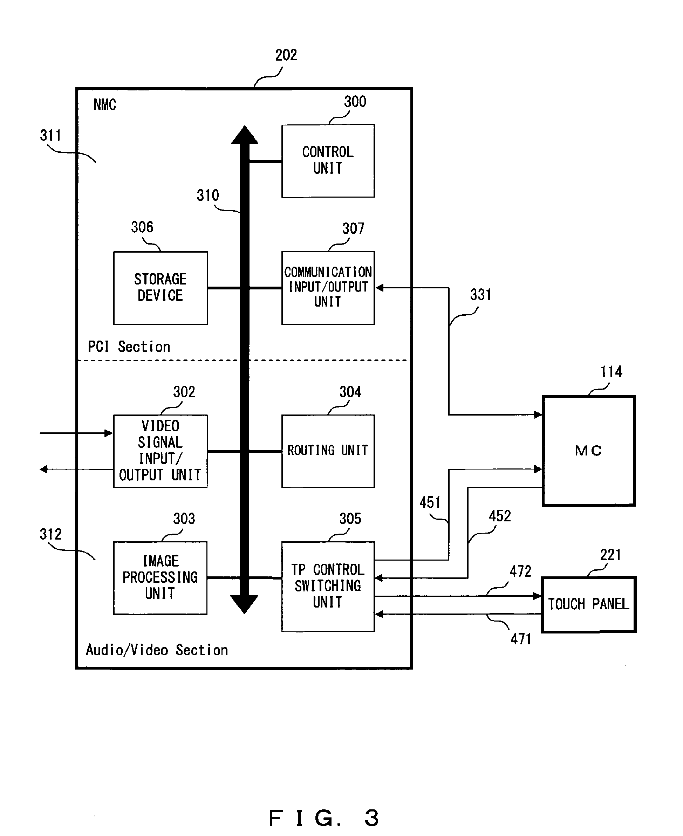

Fig. 3 is a block diagram showing a configuration of the NMC 202 in the present embodiment. The NMC 202 includes a PCI section 311 and an audio/video (A/V) section 312.

The PCI section 311 mainly controls a non-medical device group 210 connected to the NMC 202. The PCI section 311 includes a control unit 300, a storage device 306, and a communication input/output unit 307. Numerical symbol 310 denotes a back plane.

The control unit 300 controls the entirety of the PCI section 311, and transmits and receives data to and from the A/V section 312. The control unit 300 creates Graphical User Interface image information (hereinafter referred to as GUI image information) that is an image layout to be displayed on a TP 221 or a monitor device, and transmits it to a routing unit 304. Also, as will be described later, the control unit 300 performs processes for the transmission and reception of data based on communications with the MC 114 via a communication line 331. The NMC 202 and the MC 114 monitor each other via the communication line 331, and synchronize each other's GUI environment.

The storage device 306 stores various programs, information set by the TP 221, and the like. The communication input/output unit 307 is a communication interface used for the communications with the MC 114 via the communication line 331.

The A/V section 312 is a section that mainly processes the video signals and the audio signals. The A/V section 312 includes a video signal input/output unit 302, an image processing unit 303, a routing unit 304, and a TP control switching unit 305.

The video signal input/output unit 302 has a plurality of video signal input ports and a plurality of video signal output ports.

The routing unit 304 switches routes for the video signals that were processed in the image processing unit 303 and the video signals input from the video signal input/output unit 302, and transfers them to a prescribed configuration unit in the NMC 202. Also, the routing unit 304 transfers to the TP control switching unit 305 the GUI image information created in the control unit 300.

The image processing unit 303 performs image processing on the image information transferred from the routing unit 304. Examples of the image processing are enlargement/reduction (scaling) of images, mirroring of images, rotation of images, displaying another, smaller image in a main image (picture in picture (PIP)), and displaying a plurality of images simultaneously (picture out picture (POP)).

TP coordinate communication lines 451 and 471 are communication lines through which TP coordinate signals generated by touch manipulations on the TP 221 are conveyed. TP image lines 452 and 472 are image lines through which image signals such as GUI images or the like to be displayed on the TP 221 are conveyed.

The TP control switching unit 305 synthesizes the GUI image created in the control unit 300 with images created on the basis of the video signals transmitted from the video signal input/output unit 302. Then, the TP control switching unit 305 outputs the synthesized image to the TP 221. Further, the TP control switching unit 305 can perform switching between the NMC 202 and the MC 114 as the manipulation targets of the TP 221. In other words, the TP control switching unit 305 receives TP coordinate information based on the touch manipulation on the TP 221 from the TP 221, transfers the received TP coordinate information to the control unit 300, or transfers the TP coordinate information to the MC 114.

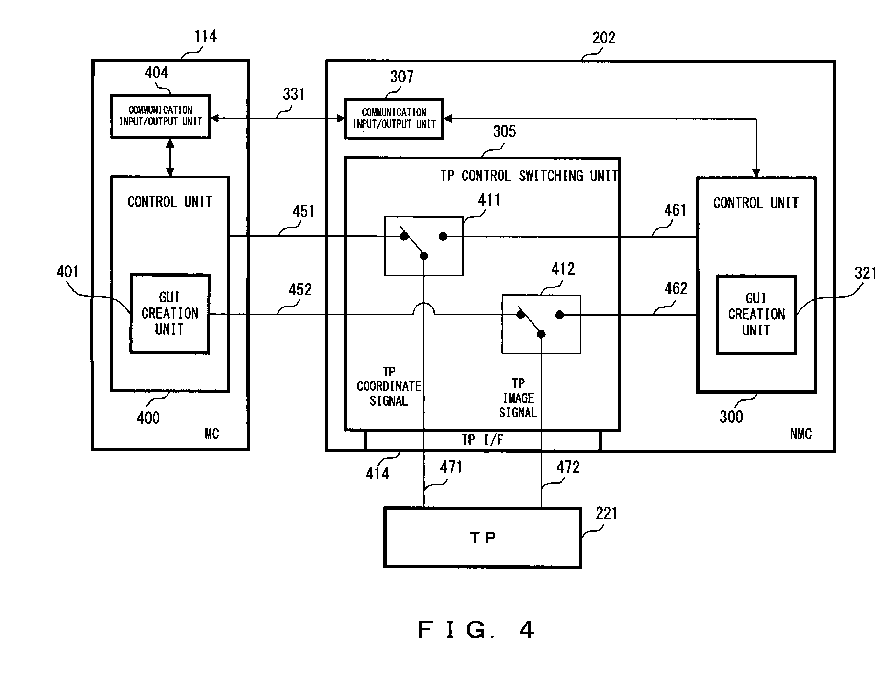

Fig. 4 shows the switching of the control targets between the NMC 202 and the MC 114 by using the TP control switching unit 305 according to the present embodiment.

As described above, the MC 114 (first controller) is a controller mainly for medical devices. The MC 114 creates a graphical user interface (first GUI) that is a window used for controlling the medical devices.

As described above, NMC 202 (second controller) is a controller mainly for non-medical devices. The NMC 202 creates another graphical user interface (second GUI)as a window used for controlling the non-medical devices.

The first and second GUIs are designed on the basis of a common graphical user interface (common GUI). For example, by switching the tabs on a window on the TP 221, the first and second GUIs can be switched therebetween.

The control unit 300 has a GUI creation unit 321. The GUI creation unit 321 creates the second GUI.

The control unit 400 has a GUI creation unit 401. The GUI creation unit 401 creates the first GUI.

TP coordinate communication lines 451, 461, and 471 are communication lines through which TP coordinate signals of the TP 221 are transmitted to the control unit 400 of the MC 114 or to the control unit 300 of the NMC 202.

TP image lines 452, 462, and 472 are image lines through which video signals of the first GUI image, the second GUI image, or the like to be displayed on the TP 221 are transmitted from the control unit 400 of the MC 114 to the TP 221 or from the control unit 300 of the NMC 202 to the TP 221.

The TP control switching unit 305 has a TP coordinate communication line switch 411, a TP image line switch 412, and a TP interface 414 (TP I/F).

The TP interface 414 is an interface to which the TP image line and the TP coordinate communication line for connecting the TP 221 and the NMC 202 are connected.

The TP coordinate communication line switch 411 is a switch device used for connecting the TP coordinate communication line 471 to the TP coordinate communication line 451 on the MC 114 side or to the TP coordinate communication line 461 on the NMC 202 side.

The TP image line switch 412 is a switch device used for connecting the TP image line 472 to the TP image line 452 on the MC 114 side or to the TP image line 462 on the NMC 202 side.

Explanation will be given for a case in which the TP coordinate communication line 451 is selected by the TP coordinate communication line switch 411 and the TP image line 452 is selected by the TP image line switch 412.

Data of the first GUI generated in the GUI creation unit 401 (TP image signal) is input into the TP 221 via the TP image line switch 412, and the first GUI is displayed on the TP 221. In this case, a TP coordinate signal 481 generated by touch manipulations on the first GUI displayed on the TP 221 is input into the control unit 400 from the TP 221 via the TP coordinate communication line switch 411.

Explanations will be given for a case in which the TP coordinate communication line 461 is selected by the TP coordinate communication line switch 411 and the TP image line 462 is selected by the TP image line switch 412. Data of the second GUI generated in the GUI creation unit 401 (TP image signal) is input into the TP 221 via the TP image line switch 412, and the second GUI is displayed on the TP 221. The TP coordinate signal 481 generated by the touch manipulations on the second GUI displayed on the TP 221 is input into the control unit 300 from the TP 221 via the TP coordinate communication line switch 411.

Next, explanations will be given for a case in which the first GUI transitions to the second GUI in response to the touch manipulations on the TP 221. The TP coordinate signal generated by the touch manipulation on the TP 221 is transmitted to the control unit 400 of the MC 114. The control unit 400, having received this TP coordinate signal, reports via the a communication input/output unit 404 and the communication line 331 to the control unit 300 of the NMC 202 the fact that the first GUI will be switched to the second GUI. The control unit 300, having received this report, controls the TP control switching unit 305 and switches the TP coordinate communication line switch 411 and the TP image line switch 412 so that the TP coordinate communication line 461 and the TP image line 462 on the NMC 202 side are enabled. Then, the control unit 300 causes the TP 221 to display the second GUI via the TP image lines 462 and 472. Additionally, this process is also applied to the case in which the second GUI transitions to the first GUI in response to the touch manipulations.

The control unit 300 and the control unit 400 communicate with and monitor each other via the communication line 331. Also, the control unit 300 and the control unit 400 synchronize each other's GUI environment, and exchange information that has to be held by both of them for configuring a common GUI. An example of the above information are window elements (window element information such as tab names) to be used in common.

As described above, the target to be controlled by the TP 221 is changed between the MC 114 and the NMC 202 on the basis of the switching operations of the TP control switching unit 305. This switching operation is not perceived by users, and accordingly users feel as if they are controlling only one controller.

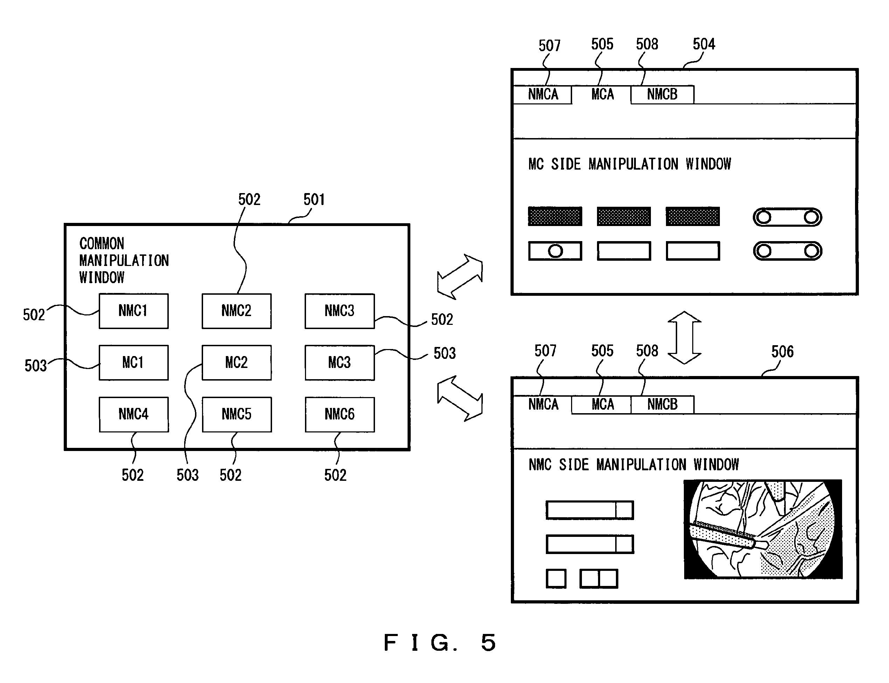

Fig. 5 shows transitions of the GUIs displayed on the TP 221. A main GUI 501 shown in Fig. 5 is a common manipulation window used for selecting the first or second GUI. By performing touch manipulations on the TP 221, the first or second GUI created in the MC 114 and the NMC 202 is selected. For example, when functions on the NMC 202 side are to be used, NMC selection switch buttons 502 (NMC 1 through NMC 6) are used, and when functions on the MC 114 side are to be used, MC selection switch buttons 505 (MC 1 through MC 3) are used. The main GUI 501 may be created on the MC 114 side, and also may be created on the NMC 202 side.

When one of the MC selection switch buttons on the main GUI 501 is selected, the first GUI (manipulation window on the MC side) is opened, and an MC-side manipulation window 504 and the like is displayed. An example of the MC-side manipulation window 504 is a window used for setting parameters or the like for medical devices. On the MC-side manipulation window 504, an NMC transition switch button 507 (NMCA) for opening an NMC-side manipulation window 506 and an NMC transition switch button 508 (NMCB) are displayed.

Also, when one of the NMC selection switch buttons 502 on the main GUI 501 is selected, the second GUI (manipulation window on the NMC side) is opened, and an NMC-side manipulation window 506 or the like is displayed. An example of the NMC-side manipulation window 506 is a window used for setting parameters or the like for non-medical devices. On the NMC-side manipulation window 506, an MC transition switch button 505 (MCA) used for opening the MC-side manipulation window 504 is displayed.

Also, when it is desired that the MC-side manipulation window 504 transits to the NMC-side manipulation window 506, the NMC transition switch button 507 is selected.

Also, when it is desired that the NMC-side manipulation window 506 transits to the MC-side manipulation window 504, the MC selection switch button 505 is selected.

Also, in the first and second GUIs that are not shown in Fig. 5, by selecting transition switch buttons corresponding to the NMC transition switch buttons 507 and 508, first and second GUIs can be opened.

In other words, users who use the common GUI on which the first and second GUIs are displayed alternately can use the system without being conscious of the fact that the GUIs that are created by different controllers are switched therebetween.

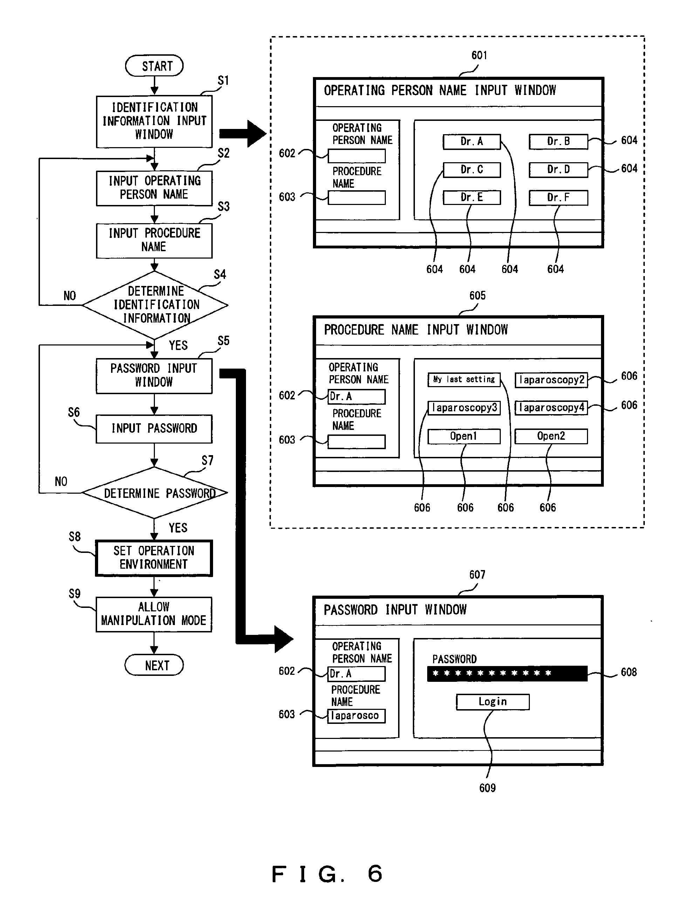

By referring to Fig. 6, log-in to the medical support control system for controlling the above described MC 114 and the NMC 202 is explained.

In step S1, an identification information input window for receiving identification information is displayed on the TP 221. Identification information input windows 601 and 605 have an operating-person-name input box 602 and a procedure-name input box 603. The identification information includes at least an operating person and a procedure name.

In step S2, an operating person is input. On the identification information input window 601 (operating-person-name input window), the operating person name is input directly into the operating-person-name input box 602 or the operating person name is selected from among the registered operating person names. In this example, operating-person-name selection switch buttons 604 (Dr. A through Dr. F) are prepared as a list of the operating persons, and the operating person is selected from among the operating-person-name selection switch buttons 604.

In step S3, a procedure name is input. On the identification information input window 605 (procedure-name input window), a procedure name is input directly into the procedure-name input box 603 or the procedure name is selected from among the registered procedure names. In this example, procedure name selection switch buttons 606 ("My last setting", "laparoscopy2", "laparoscopy4", "laparoscopy3", "Open1", "Open2", and the like) are prepared as the procedure names, and one of them is selected.

In step S4, it is determined whether or not the identification information has been input correctly. For example, it is determined whether or not the input operating person name or the input procedure name involves an error. When an error is involved, the process proceeds to step S2. When the identification information is correct, the process proceeds to step S5.

In step S5, a password input window 607 is displayed for accepting passwords. The password input window 607 has the operating-person-name input box 602, the procedure-name input box 603, a password input box 608, and a log-in button 609.

In step S6, a password is input. One password is prepared for each piece of identification information input in the above step.

In step S7, it is determined whether or not the input password is correct. It is determined whether or not the password corresponding to the identification information has been input. When the corresponding password has been input, the process proceeds to step S8, and the operation setting is performed. When the correct password has not been input, the process proceeds to step S5, and a password is again input.

In step S8, operation environment setting (that will be described later) is performed.

In step S9, a manipulation mode starts. The "manipulation mode" is a mode in which medical devices enter into an operation state.

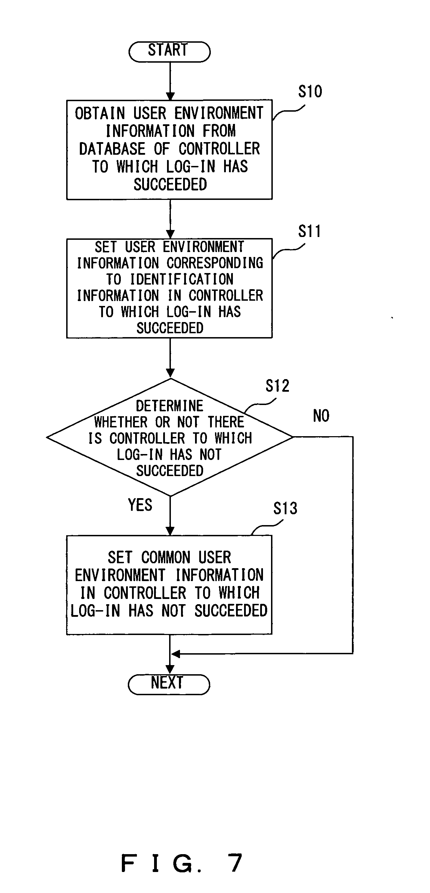

Fig. 7 is a flowchart for setting the operation environment.

In step S10, user environment information is obtained from the first or second controller to which log-in has succeeded. For example, when the log-in to the MC 114 and the NMC 202 has been successful, the user environment information is obtained respectively from the MC 114 and the NMC 202.

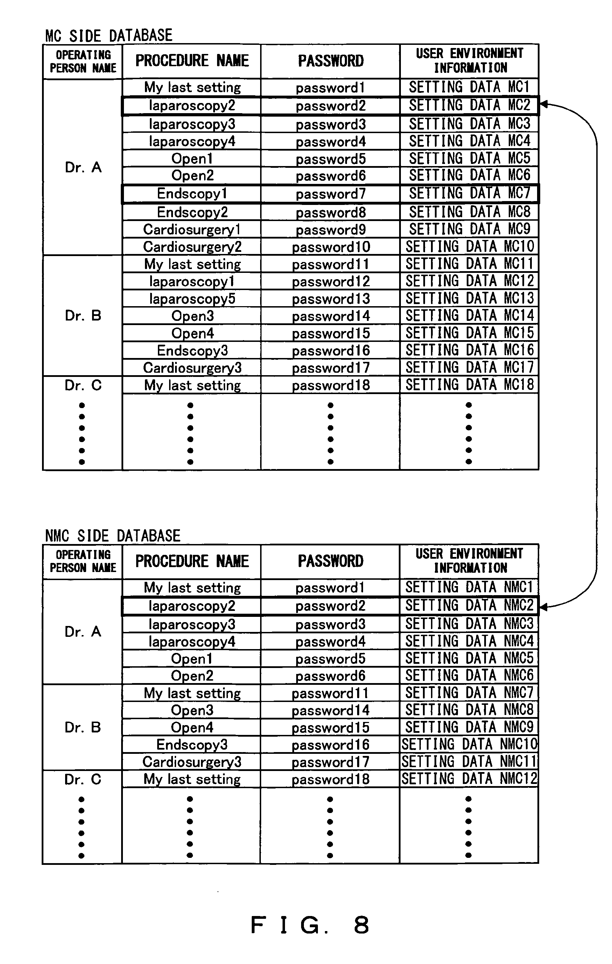

For example, user environment information corresponding to the identification information is obtained by using an MC-side database provided in the MC 114 and an NMC-side database provided in the NMC 202, as shown in Fig. 8. When the operating person is "Dr. A", the procedure name is "laparoscopy2", and the password is "password2", "setting data MC2" and "setting data NMC2" are obtained (arrow). The MC-side database is stored in a storage unit in the MC 114, and the NMC-side database is stored in the storage unit 306 or the like.

Also, when there is no identification information that is included only in the MC-side database shown in Fig. 8 in the case when log-in is only successful to one of the controllers, the user environment information corresponding in the MC-side database is obtained. The operation environment in which the operating person is "Dr. A", the procedure name is "endscopy1", and the password is "password7" is selected and only "setting data MC7" is obtained.

Also, there is a case in which identification information that is included only in the NMC-side database is selected.

In step S11, the user environment information corresponding to the identification information is set in the controller to which log-in has succeeded. Different pieces of user environment information are set in the MC 114 and the NMC 202.

For example, when the operating person is "Dr. A", the procedure name is "laparoscopy2", and the password is "password2", settings that are appropriate to the medical devices required for performing the laparoscopy examination on the MC 114 side are set in accordance with the contents of "setting data MC2". Also, settings that are appropriate to the non-medical devices required for performing the laparoscopy examination on the NMC 202 are performed in accordance with the contents of the "setting data NMC2".

In step S12, it is determined whether or not there is a controller to which log-in has not succeeded, and when there is, the process proceeds to step S13. For example, a case in which log-in was only successful to the MC 114 side or a case in which log-in was only successful to the NMC 202 side is detected.

In step S13, common user environment information is set in a controller to which log-in has not succeeded. The common user environment information is, for example, the initial values of parameters for medical devices and non-medical devices. It is also possible to set appropriate parameters for each medical device or non-medical device in advance, and to use such parameters as the common user environment information.

As described above, it is possible to provide a medical support control system for controlling medical devices and non-medical devices.

Conventionally, when it is desired to control a plurality of controllers in a system, log-in to each controller has been required. However, in the above configuration, by only logging in to one controller, it is possible to log in to the other controllers, which simplifies operations.

Also, if the respective controllers have the same identification information at the time of log-in, the operation environments for the controllers can be set by reading user environment information stored in each controller by using the identification information.

Even when there is a controller that cannot be logged into, the controller that cannot be logged into is logged into by using a general environment (common user environment information), and thereby operations are simplified.

The scope of the present invention is not limited to any of the above embodiments, and various alterations and modifications are allowed without departing from the spirit of the present invention.

### <The second embodiment>

In the present embodiment, explanations are given to a medical support control system in which, even when the subject of controlling the medical support control system is the first controller, if a prescribed event occurs, it is possible to report to the users the occurrence of the event and to switch the controlling subject to the second controller.

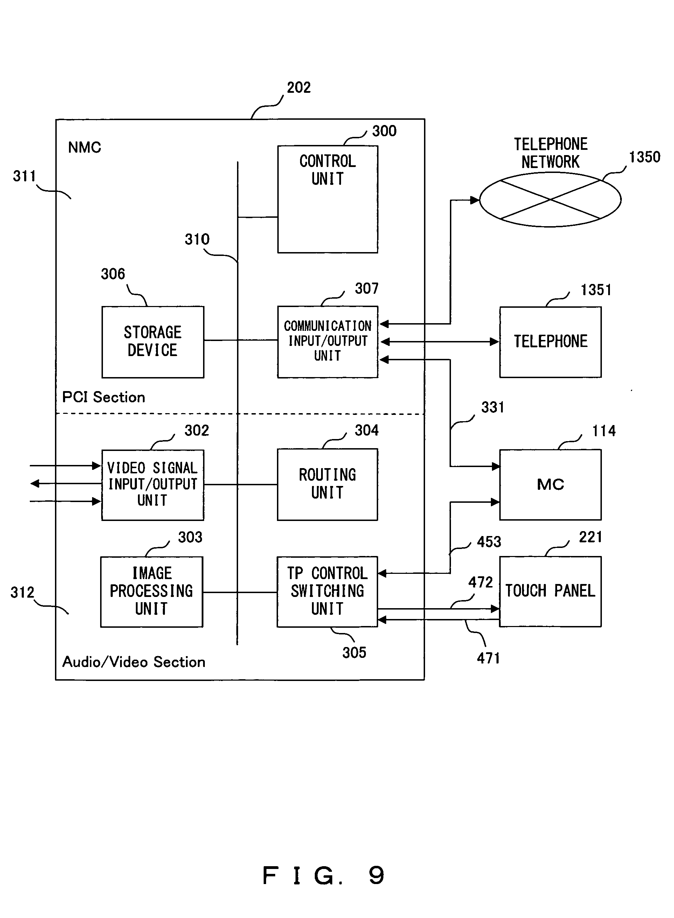

Fig. 9 is a block diagram showing a configuration of the NMC 202 in the second embodiment. In the second embodiment, The communication input/output unit 307 is connected to a telephone circuit 1350 and a communication network such as a Local Area Network (LAN) or the like in addition to the MC 114. TP control signal line 453 is corresponding to TP coordinate communication lines 451 and TP coordinate communication line 451.

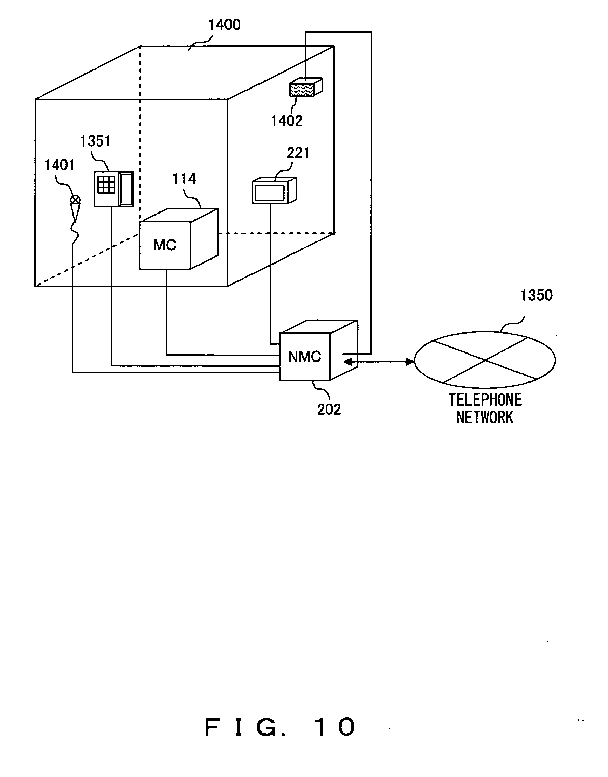

Fig. 10 shows an example of a setting environment for the MC 114 and the NMC 202 according to the present embodiment. The NMC 202 is provided in a place out of an operation room 1400, and is connected to the telephone circuit 1350 in the present embodiment.

The MC 114 is provided in the operation room 1400. Also, the operation room 1400 has a microphone 1401, a speaker device 1402, a telephone 1351, and the TP 221 that are connected to the NMC 202.

Also, the location for the NMC 202 is not limited to a place outside of the operation room 1400, but may be located in the operation room 1400.

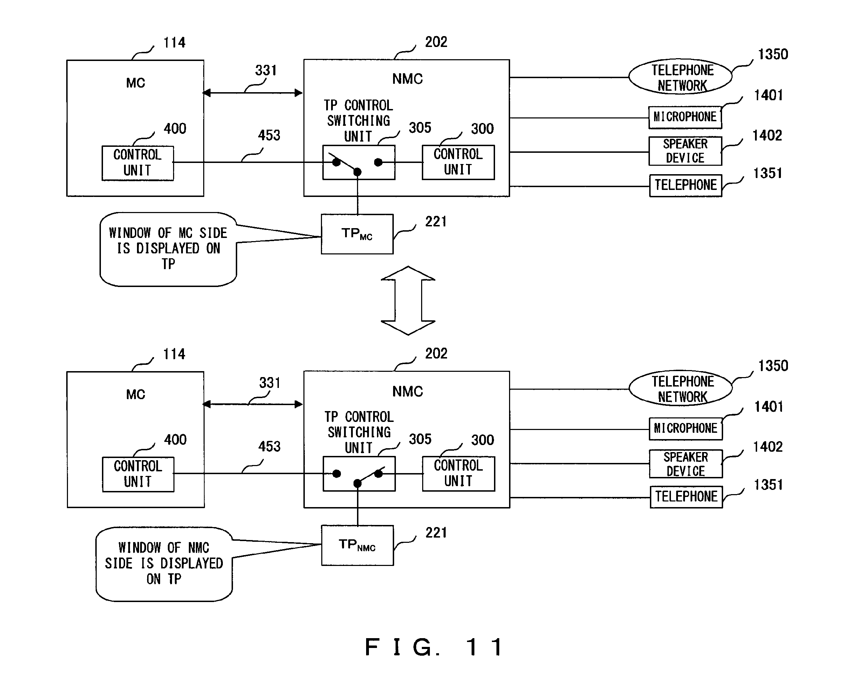

Fig. 11 shows switching operation of the control target of the TP control switching unit 305 performed between the NMC 202 and the MC 114.

As described above, the MC 114 is a controller mainly for medical devices. The MC 114 creates a graphical user interface (first GUI) that is a window used for controlling the medical devices belonging to the MC 114 itself.

As described above, the NMC 202 is a controller mainly for non-medical devices. The NMC 202 creates a graphical user interface (second GUI) that is a window used for controlling the non-medical devices belonging to the NMC 202 itself.

The first and second GUIs are designed on the basis of a common graphical user interface (common GUI). For example, by switching the tabs in a window, the first and second GUIs can be switched therebetween. This operation will be explained later in detail.

The MC 114 and the NMC 202 are connected to each other via the communication line 331 used for transmitting and receiving data to and from each other and via the TP control signal line 453 used for manipulating the MC 114 by using the TP 221 via the NMC 202.

When the first GUI is displayed on the TP 221, if the second GUI is to be opened via touch manipulation, a control signal (TP control signal) activated via touch manipulation is transmitted to the control unit 400 of the MC 114. The control unit 400, having received the TP control signal, reports, to the control unit 300 of the NMC 202, the fact that the first GUI is switched to the second GUI via a communication line 331. The control unit 300, having received this report, controls the TP control switching unit 305, and switches the TP control signal line from the MC 114 side to the NMC 202 side. Then, the control unit 300 causes the TP 221 to display the second GUI. Also, the same operation is performed if the first GUI is opened by touch manipulation when the second GUI is displayed on the TP 221.

As described above, the control unit 300 and the control unit 400 communicate with and monitor each other via the communication line 331. Also, the control unit 300 and the control unit 400 synchronize each other's GUI environment via the communication line 331, and exchange information to be held in common for configuring a common GUI (for example, constituent elements (window item information such as tab name or the like) for windows to be used in common).

In Fig. 11, when switching is performed for the MC 114 side by the TP control switching unit 305 (upper view), a window controlled by the MC 114 is displayed on the TP 221. Then, a touch signal of the TP 221 is transmitted to the control unit 400 of the MC 114 via the TP control switching unit 305 and the TP control signal line 453. In this case, the TP 221 displays the first GUI created by the MC 114, and by using this first GUI, MC 114 can be controlled. The touch panel in the case when the MC 114 is controlled by using the first GUI is denoted by TP_{MC}.

In Fig. 11, when switching is performed for the NMC 202 side by the TP control switching unit 305 (lower view), a window controlled by the NMC 202 is displayed on the TP 221. Then, the TP control signal created by manipulating the TP 221 is transmitted to the control unit 300 of the NMC 202 via the TP control switching unit 305. In this case, the second GUI created by the NMC 202 is displayed, and by using this GUI, the NMC 202 can be controlled. The touch panel in the case when the NMC 202 is controlled by using the second GUI is denoted by TP_{NMC}.

By employing the above configuration, the target controlled by the TP 221 is switched between the MC 114 and the NMC 202 by using the TP control switching unit 305. However, this switching is not perceived by users, and accordingly users feel as if they have controlled only one controller.

As described above, the TP 221 connected to the NMC 202 is shared by the MC 114 and the NMC 202. Also, the first GUI created in the MC 114 and the second GUI created in the NMC 202 are displayed in the common GUI format on the TP 221.

When the first GUI is displayed on the TP 221 in the present embodiment, if the NMC 202 receives a call via the telephone circuit 1350, a symbol indicating that there is a telephone call is displayed on the TP 221. When this symbol is selected and pressed, the window is switched to a telephone conversation window of the second GUI, and the telephone conversation is enabled via the NMC 202.

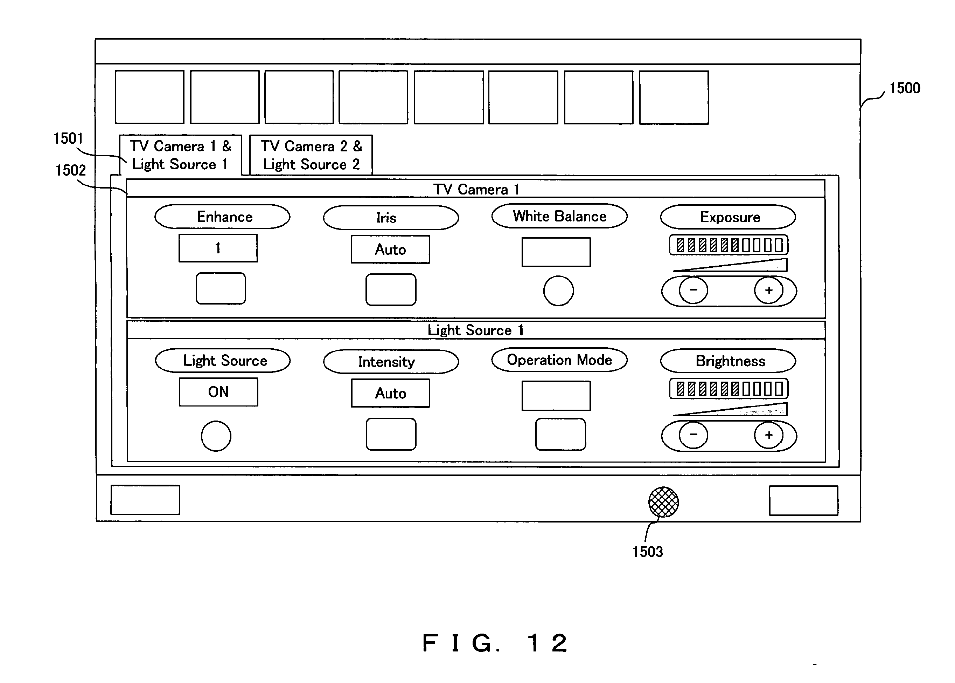

Fig. 12 shows an example of a display window (first GUI) of the touch panel (TP_{MC}) when the MC 114 side is the control target. In a window 1500, shown in Fig. 12, a tab "TV Camera 1 and Light Source 1" 1501 is selected as an example, and a selection tab display area 1502 is displayed. Thus, on the selection tab display area 1502, it is possible to adjust "TV camera 1" and "Light Source 1".

Also, in a lower portion of the window 1500, a prescribed symbol 1503 is displayed. This symbol 1503 is not usually displayed in a window. When there is a phone call to the NMC 202, the symbol 1503 is displayed, and it is possible to report, to the users viewing the TP 221, the fact that there is a telephone call to the NMC 202. Also, the symbol 1503 is not limited as long as it can visually report to the users the fact of a telephone call. Also, the symbol 1503 may be displayed on the TP 221 in an emphasized manner such as by flashing or by being enlarged or reduced. Also, the symbol 1503 may be arranged at any position in the window.

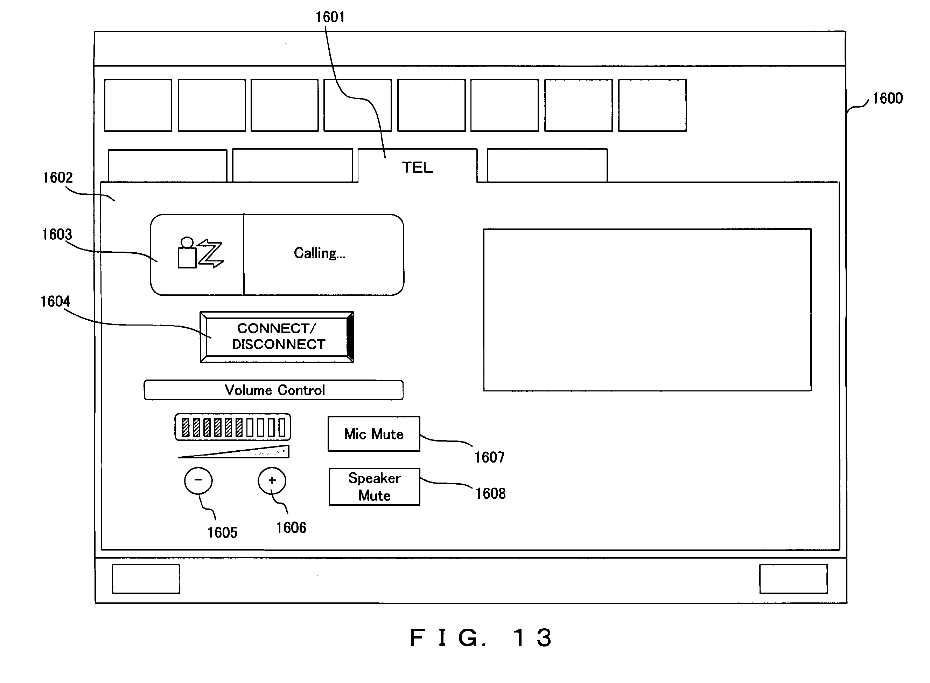

Fig. 13 shows an example of a telephone conversation window (second GUI) of the touch panel (TP_{NMC}) displayed when the NMC 202 side is the control target. In a window 1600, shown in Fig. 13, a tab "TEL" 1601 is selected as an example, and a selection tab detailed display area 1602 is displayed.

The selection tab detailed display area 1602 has a telephone conversation state display area 1603, a connection/disconnection button 1604, volume control buttons 1605 and 1606, a microphone mute button 1607, and a speaker mute button 1608.

On the telephone conversation state display area 1603, telephone conversation states such as "calling", "during conversation", or "conversation is ended" are displayed. When the connection/disconnection button 1604 is pressed during a conversation, the connection can be disconnected. When the connection/disconnection button 1604 is pressed when the line is not connected, it is possible to make a call.

When the volume control button 1605 is pressed, the volume of the voices output from the speaker device 1402 can be reduced. When the volume control button 1606 is pressed, the volume of the voices output from the speaker device 1402 can be increased. When the microphone mute button 1607 is pressed, it is possible to mute the voices to be input into the microphone 1401. When the speaker mute button 1608 is pressed, it is possible to mute the voices output from the speaker device 1402.

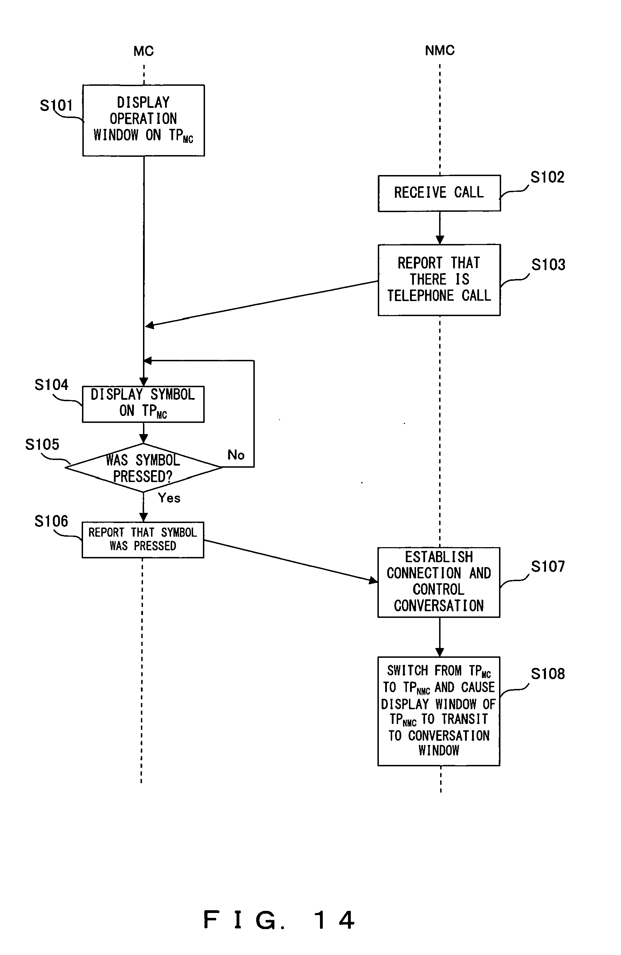

Fig. 14 is a flowchart for switching the control target between the MC 202 and the MC 114, performed by the TP control switching unit 305. First, a window controlled by the MC 114 is displayed on the TP_{MC} (as in Fig. 12) (S101). In this case, the TP control switching unit 305 is in the state shown in the upper view of Fig. 11.

When there is a telephone call to the NMC 202 from an external environment in the above case (S102), the control unit 300 reports this fact to the MC 114 via the communication line 331 (S103).

The control unit 400 of the MC 114, having received this report from the NMC 202, displays the symbol 1503 on a lower portion of the TP_{MC} (S104). When the symbol 1503 is pressed as one of the touch manipulations by the users ("Yes" in S105), the control unit 400 reports this fact to the NMC 202 via the communication line 331 (S106).

The control unit 300, having received the report from the MC 114, establishes the connection for the call and performs control for enabling the conversation (S107). Then, the TP control switching unit 305 switches the target controlled by the TP 221 from the MC 114 to the NMC 202 according to an instruction of the control unit 300. In this case, the TP control switching unit 305 is in the state shown in the lower view in Fig. 11. Further, the NMC 202 causes the TP_{NMC} to display the conversation window (Fig. 13). Then, the user can have a conversation via the microphone 1401 and the speaker device 1402.

In the conventional techniques, it has been impossible to answer a call when a window created in the MC 114 is being displayed on the TP 221. However, according to the present embodiment, it is possible to answer a call by pressing the symbol 1503 on the TP 221 even when a window created by the MC 114 is being displayed on the TP 221.

When an e-mail is received from a network to which the NMC 202 is connected while the GUI on the MC 114 side is being displayed on the TP 221, it is impossible to open the e-mail immediately. The present invention enables users to open the e-mail even in such cases.

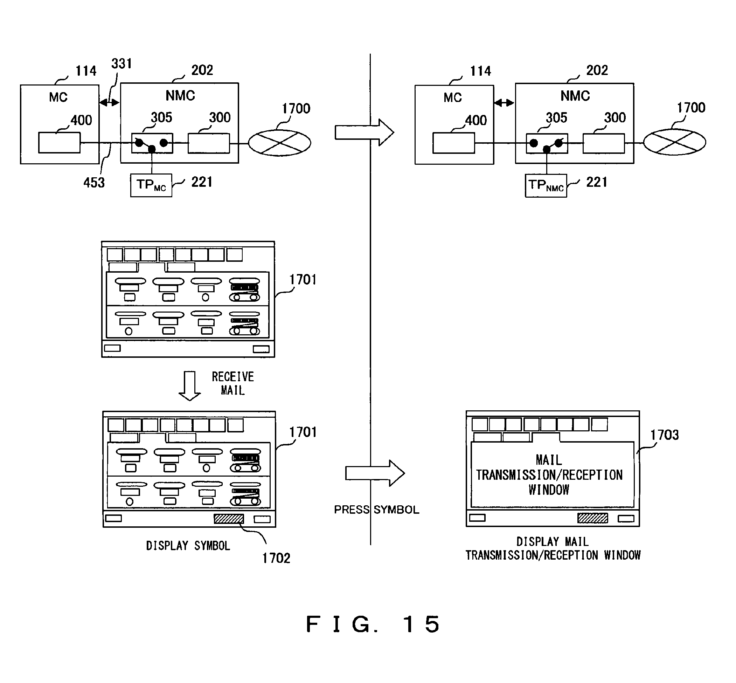

Fig. 15 shows a switching operation from a window of the MC 114 side to a window of the NMC 202 side performed when e-mails are received while the window of the MC 114 side is being displayed according to the present embodiment (variation 1). In the variation 1, the NMC 202 is connected to a network 1700 such as a local area network (LAN), a wide area network (WAN), the Internet, an intranet, or the like.

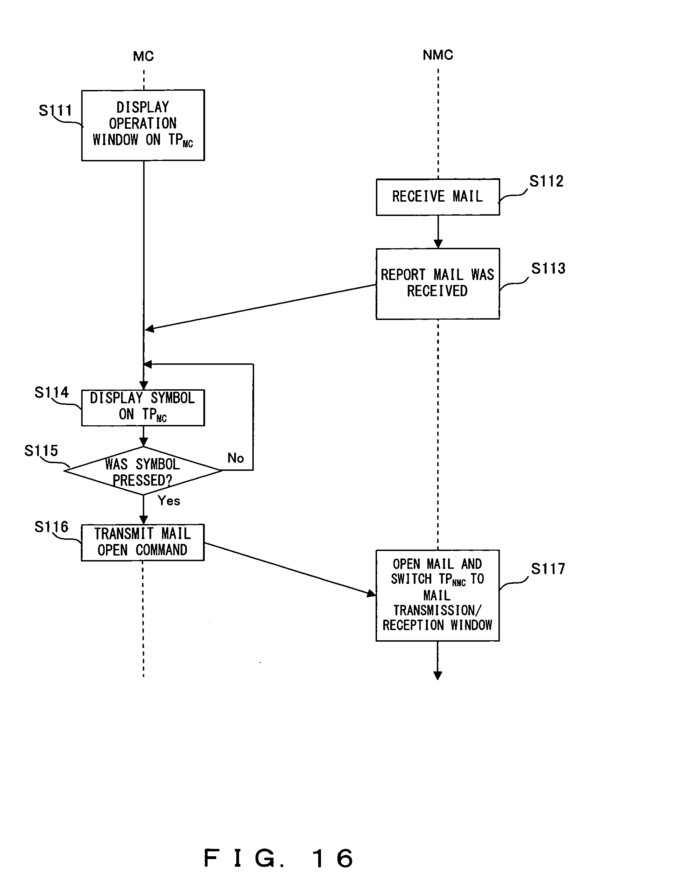

Fig. 16 is a flowchart for switching a control target between the NMC 202 and the MC 114 performed by the TP control switching unit 305 according to the present embodiment (variation 1). Hereinafter, explanations will be given by referring to Figs. 15 and 16.

First, a window 1701 to be controlled by the MC 114 is displayed on the TP_{MC} (S111). The NMC 202 receives an e-mail via the network 1700 (S112). Then, the control unit 300 reports to the MC 114 via the communication line 331 the fact that an e-mail has been received (S113).

The control unit 400, having received the report from the NMC 202, displays a "mail" button 1702 as a symbol in the lower part of a window TP_{MC} (S114). When the "mail" button 1702 is pressed as one of the user's manipulations (Yes in S115), the control unit 400 transmits a mail open command to the NMC 202 via the communication line 331 (S116).

The TP control switching unit 305, having received the command from the MC 114, switches the target to be controlled by the TP 221 from the MC 114 to the NMC 202. Further, the NMC 202 causes the TP_{NMC} to display a mail transmission/reception window 1703, and opens the received e-mail (S117).

Thereby, when the NMC 202 has received an e-mail, it is possible to open the e-mail even when the GUI of the MC 114 side is being displayed on the TP 221.

Also, when there is no more free space in a storage medium (such as video tape, a DVD, a Blu-ray disk, an HD DVD, flash memory, a hard disk, or the like) used for recording an image while a GUI of the MC 114 side is being displayed on the TP 221, it is impossible to replace the storage medium with a new one immediately. It is desired that storage media be able to be replaced in such a case too.

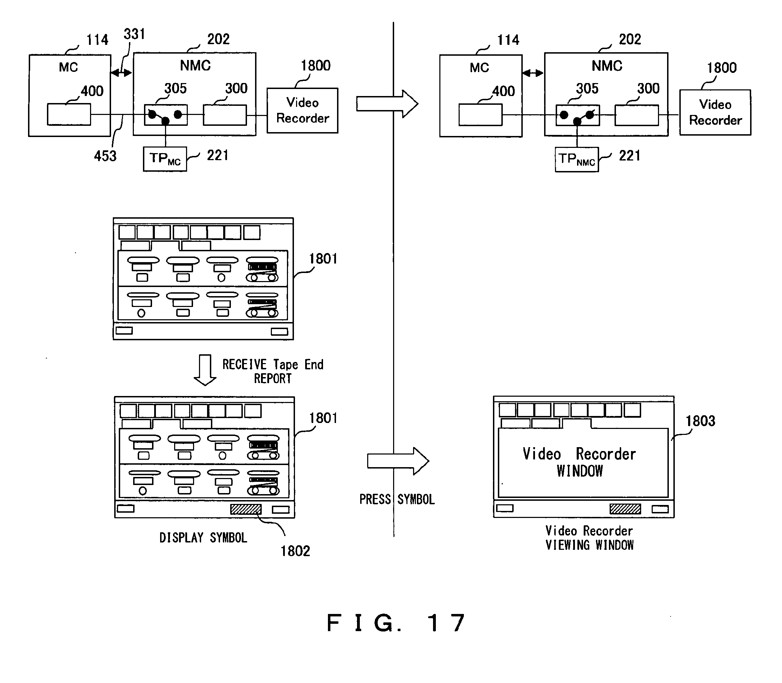

Fig. 17 shows the switching to a window of the NMC 202 side performed when there is no more free space in a storage medium while a window of the MC 114 side is being displayed according to the present embodiment (variation 2). In variation 2, a video recorder 1800 is connected to the NMC 202.

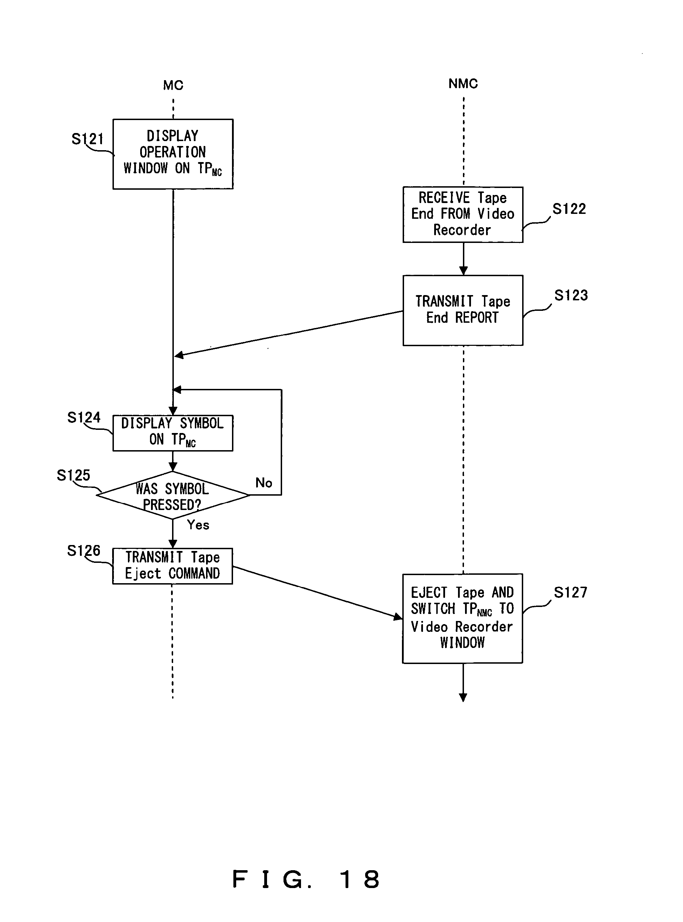

Fig. 18 is a flowchart for switching a control target between the NMC 202 and the MC 114, performed by the TP control switching unit 305 according to the present embodiment (variation 2). Hereinafter, explanations will be given by referring to Figs. 17 and 18. In the example below, tape is used as a storage medium; however, any type of storage medium including a DVD, a Blu-ray disk, an HD DVD, flash memory, a hard disk, or the like can be used.

First, a window 1801 to be controlled by the MC 114 is displayed on the TP_{MC} (S121). The NMC 202 receives from the video recorder 1800 a report indicating that the tape has reached its end (a tape-end report) (S122). Then, the control unit 300 transmits to the MC 114 via the communication line 331 the tape-end report (S123).

The control unit 400, having received the report from the NMC 202, displays a "Tape End" button 1802 as a symbol in a lower part of the window of the TP_{MC} (S124). When the "Tape End" button 1802 is pressed as one of the user's manipulations (Yes in S125), the control unit 400 transmits to the NMC 202 via the communication line 331 a tape-eject command (S126).

The control unit 300, having received the command from the MC 114, causes the video recorder 1800 to eject the video tape. The TP control switching unit 305 switches the target to be controlled by the TP 221 from the MC 114 to the NMC 202. Then, the NMC 202 causes the TP_{NMC} to display a video recorder window 1803 (S127).

Thereby, when there is no more free space in a storage medium while an image is being recorded in the storage medium using a video recorder connected to the NMC 202, it is possible to easily replace the storage medium with a new one even when a GUI of the MC 114 side is being displayed on the TP 221. Also, an example of a video recorder is used in variation 2; however, variation 2 can be applied to other devices. Further, in variation 2, events such as displaying a symbol or switching controllers occur in response to the report indicating that there is no more free space in a storage medium; however, events such as displaying a symbol or switching controllers or the like can occur in response to errors in devices or a warning message transmitted from devices.

According to the present embodiment, the medical support control system includes a first controller (MC 114), a second controller (NMC 202), and a manipulation display device (TP 221). The first controller is connected to at least one device. The second controller is connected to at least one device and to a network, and is provided in a medical environment different from that of the first controller. On the manipulation display device, a first graphical user interface (first GUI) that is shared by the first and second controllers and is created by the first controller and a second graphical user interface (second GUI) created by the second controller are displayed alternately. Thesecond controller has a switching unit for switching the GUI to be displayed on the manipulation display device between the first and second GUIs. The switching unit switches the GUI to be displayed on the manipulation display device from the first GUI to the second GUI on the basis of the report from the first controller indicating that the symbol has been pressed.

When a prescribed report is received via the network or from the prescribed device while the first GUI is being displayed on the manipulation display device, a symbol indicating the reception of the report is displayed on the first GUI. When the symbol is pressed, the second GUI corresponding to the report is displayed. Also, a process corresponding to the report is performed by the second controller.

By this configuration, even when the subject of controlling the medical support control system is the first controller, if a prescribed event occurs, it is possible to report to the users the occurrence of the event and to switch the controlling subject to the second controller.

According to one example of the present embodiment, the network is a telephone circuit. When there is a telephone call via the telephone circuit while the first GUI is being displayed on the manipulation display device, a symbol indicating that there is a telephone call is displayed on the first GUI. When the symbol is pressed, the conversation window of the second GUI is opened. Also, the conversation is enabled via the second controller.

By the above configuration, even when a window created by the MC 114 is being displayed on the TP 221, it is possible to answer a call by pressing the symbol 1503 displayed on the TP 221.

Also, according to one example of the present embodiment, when an e-mail is received via the network while the first GUI is being displayed on the manipulation display device, a symbol indicating that the e-mail has been received is displayed on the first GUI. When this symbol is pressed, an e-mail transmission/reception window of the second GUI is opened. Also, the received e-mail is displayed in the e-mail transmission/reception window.

By the above configuration, when the NMC 202 has received an e-mail, the e-mail can be opened even when the GUI of the MC 114 side is being displayed on the TP 221.

Also, according to one example of the present embodiment, the prescribed device is a video recorder. When a report is received indicating that there is no more free space in a storage medium from the video recorder while the first GUI is being displayed on the manipulation display device, a symbol indicating the reception of such a report is displayed on the first GUI. When this symbol is pressed, a video recorder manipulation window of the second GUI corresponding to the report is opened. Also, the second controller causes the video recorder to replace the storage medium with a new one.

By the above configuration, even when there is no more free space in a storage medium that is recording an image by using a video recorder connected to the NMC 202, it is possible to easily replace the storage medium with a new one even if the GUI of the MC 114 side is being displayed on the TP 221.

The scope of the present invention is not limited to the above embodiments, and various alterations and modifications are allowed without departing from the spirit of the present invention.

As described above, it is possible to provide a medical support control system that controls a medical device and a non-medical device.

### <The third embodiment>

In the present embodiment, explanations are give to a medical support control system in which first error information obtained from first controller and second error information obtained from second controller are shared by first and second controllers, and thereby not only can errors caused in the controller that is the control target of the TP 221 be displayed, but errors caused in the controller that is not the control target of the TP 221 can also be displayed.

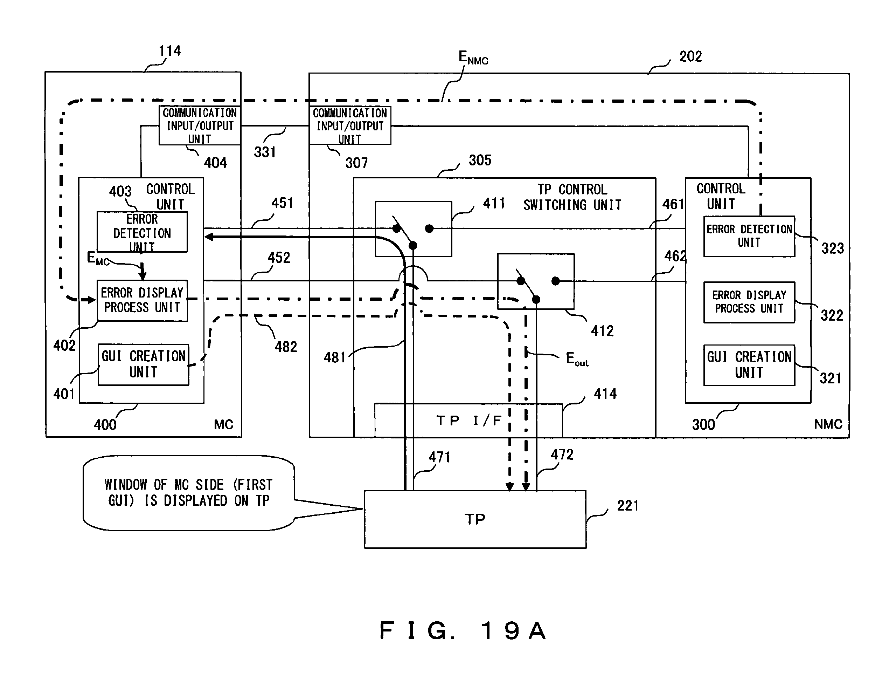

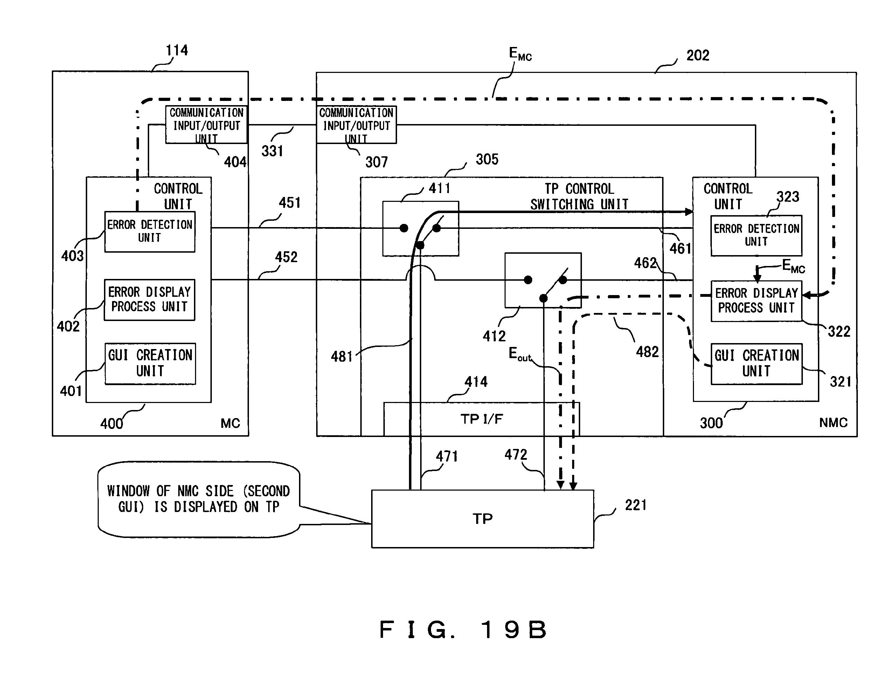

Figs. 19A and 19B respectively show the switching of the control targets between the NMC 202 and the MC 114 by using the TP control switching unit 305.

As described above, the MC 114 is a controller mainly for the medical devices. The MC 114 creates a graphical user interface (first GUI) that is a window used for controlling the medical devices.

As described above, the NMC 202 is a controller mainly for the non-medical devices. The NMC 202 creates a graphical user interface (second GUI) that is a window used for controlling the non-medical devices.

The first and second GUIs are designed on the basis of a common graphical user interface (common GUI). For example, by switching the tabs on a window on the TP 221, the first and second GUIs can be switched there between.

The control unit 300 has a GUI creation unit 321, an error display process unit 322, and an error detection unit 323. The GUI creation unit 321 creates the second GUI.

The error detection unit 323 detects error information E_{NMC} caused on the NMC 202 side. The error information E_{NMC} is error information about, for example, devices connected to the NMC 202 and is error information caused in the NMC 202.

The error display process unit 322 obtains the error information E_{NMC} caused on the NMC 202 side and the error information E_{MC} caused on the MC 114 side, and outputs to the TP 221 error information Eₒᵤₜ determined on the basis of a process flow (which will be described later).

The control unit 400 has a GUI creation unit 401, an error display process unit 402, and an error detection unit 403. The GUI creation unit 401 creates a first GUI.

The error detection unit 403 detects error information E_{MC} on the MC 114 side. The error information E_{MC} is error information about, for example, devices connected to the MC 114 and is error information caused in the MC 114.

The error display process unit 402 obtains the error information E_{NMC} caused on the NMC 202 side and the error information E_{MC} caused on the MC 114 side, and outputs to the TP 221 the error information Eₒᵤₜ determined on the basis of the process flow (which will be described later).

The TP coordinate communication lines 451, 461, and 471 are communication lines for transmitting TP coordinate signals from the TP 221 to the control unit 400 of the MC 114 or to the control unit 300 of the NMC 202.

The TP image lines 452, 462, and 472 are image lines for transmitting video signals such as a first or second GUI image to be displayed on the TP 221 from the control unit 400 of the MC 114 to the TP 221 or from the control unit 300 of the NMC 202 to the TP 221. Also, the error information Eₒᵤₜ is also output via the TP image lines 452, 462, and 472.

The TP control switching unit 305 has a TP coordinate communication line switch 411, a TP image line switch 412, and a TP I/F 414.

The TP I/F 414 is an interface to which the TP image line and the TP coordinate communication line between the TP 221 and the NMC 202 are connected.

The TP coordinate communication line switch 411 is a switch that determines whether the TP coordinate communication line 471 is to be connected to the TP coordinate communication line 451 of the MC 114 side or the TP coordinate communication line 461 of the NMC 202 side.

The TP image line switch 412 is a switch that determines whether the TP image line 472 is to be connected to the TP image line 452 of the MC 114 side or the TP image line 462 of the NMC 202 side.

A communication input/output unit 404 is a communication interface used for performing communications with the NMC 202 via the communication line 331.

Explanations will be given for a case, shown in Fig. 19A, in which the TP coordinate communication line 451 is selected by the switch 411 and the TP image line 452 is selected by the switch 412. A TP image signal 482 created by the GUI creation unit 401 is input into the TP 221 via the switch 412, and the first GUI is displayed on the TP 221. In this case, a TP coordinate signal 481 caused by touch manipulations on the first GUI displayed on the TP 221 is input into the control unit 400 from the TP 221 via the switch 411.

In the case shown in Fig. 19A, the error detection unit 403 detects the error information E_{MC} caused on the MC 114 side, and reports the error information E_{MC} to the error display process unit 402. Also, the error detection unit 323 detects the error information E_{NMC} caused on the NMC 202 side, and reports the error information E_{NMC} to the error display process unit 402 via the communication line 331. In this case, "error information Eg" represents the error information E_{MC} and the error information E_{NMC} obtained by the error display process unit 402. The error display process unit 402 determines which error information has the highest priority from among the error information Eg on the basis of the priority order set in advance, and outputs the information determined to have the highest priority as information Eₒᵤₜ to the TP 221. On the TP 221, the error information Eₒᵤₜ is displayed. If there are a plurality of pieces of information that have the highest priority, such pieces of information are displayed on the TP 221 sequentially and repeatedly at constant time intervals.

Next, explanations will be given for a case, shown in Fig. 19B, in which the TP coordinate communication line 461 is selected by the switch 411, and the TP image line 462 is selected by the TP image line switch 412. The TP image signal 482 created by the GUI creation unit 401 is input into the TP 221 via the switch 412, and the second GUI is displayed on the TP 221. The TP coordinate signal 481 caused by the touch manipulations on the second GUI displayed on the TP 221 is input from the TP 221 into the control unit 300 via the switch 411.

In the case shown in Fig. 19B, the error detection unit 323 detects the error information E_{NMC} caused on the NMC 202 side, and reports the error information E_{NMC} to the error display process unit 402. Also, the error detection unit 403 detects the error information E_{MC} caused on the MC 114 side, and reports the error information E_{MC} to the error display process unit 322 via the communication line 331. In this case, "error information E_{g}" represents the error information E_{MC} and the error information E_{NMC} obtained by the error display process unit 322. The error display process unit 322 determines which error information has the highest priority from among the error information Eg on the basis of the priority order set in advance, and outputs the information determined to have the highest priority as information Eₒᵤₜ to the TP 221. On the TP 221, the error information Eₒᵤₜ is displayed. If there are a plurality of pieces of information that have the highest priority, such pieces of information are displayed on the TP 221 sequentially and repeatedly at constant time intervals.

Next, explanations will be given for a case in which the first GUI transits to the second GUI in response to the touch manipulations on the TP 221. The TP coordinate signal 481 created by the touch manipulations on the TP 221 is sent to the control unit 400 in the MC 114. The control unit 400 that has received the TP coordinate signal 481 reports, to the control unit 300 in the NMC 202 via the communication line 331, that the first GUI will be switched to the second GUI. When receiving this report, the control unit 300 controls the TP control switching unit 305, and the switches 411 and 412 are operated so that the TP coordinate communication line 461 and the TP image line 462 on the NMC 202 side are enabled. Then, the control unit 300 causes the TP 221 to display the second GUI via the TP image lines 462 and 472. Additionally, this process is also applied to the case in which the second GUI transits to the first GUI in response to the touch manipulations.

The control units 300 and 400 monitor each other via the communication line 331. Also, the control units 300 and 400 synchronize each other's GUI environment via the communication line 331, and exchange information that has to be held by both of them for configuring a common GUI. An example of the above information is window elements (window element information such as tab names) to be used commonly.

As described above, the target to be controlled by the TP 221 is changed between the MC 114 and the NMC 202 on the basis of the switching operations of the TP control switching unit 305. This switching operation is not perceived by the users, and accordingly the users feel as if they have controlled only one controller.

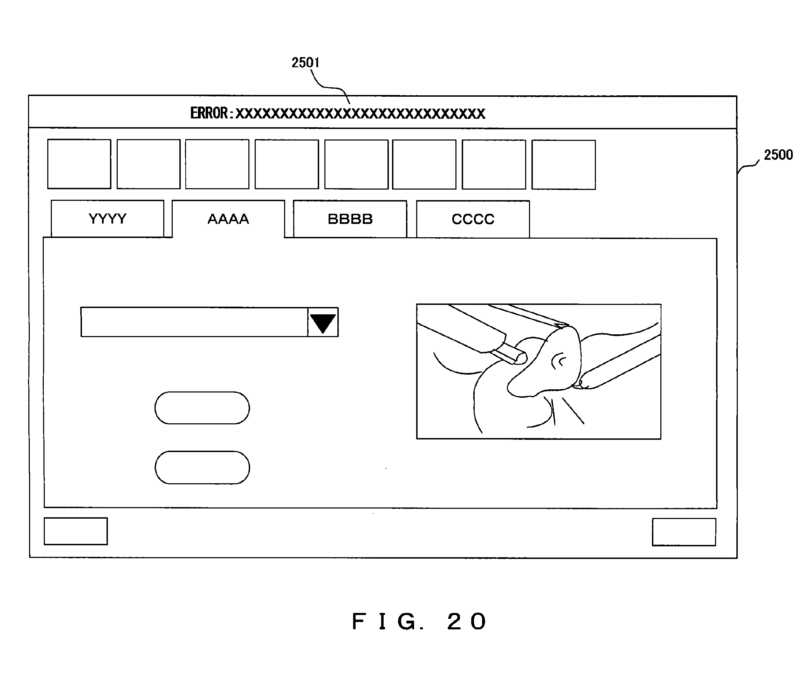

Fig. 20 shows an example of an error display displayed on an operation window according to the present embodiment. An operation window 2500 is displayed on the TP 221 on the basis of the common GUI. As explained by referring to Figs. 19A and 19B, when the error information Eₒᵤₜ is output from the MC 114 or the NMC 202, the error display is displayed in an error display area 2501 located on an upper portion of the operation window 2500 on the basis of the error information Eₒᵤₜ.

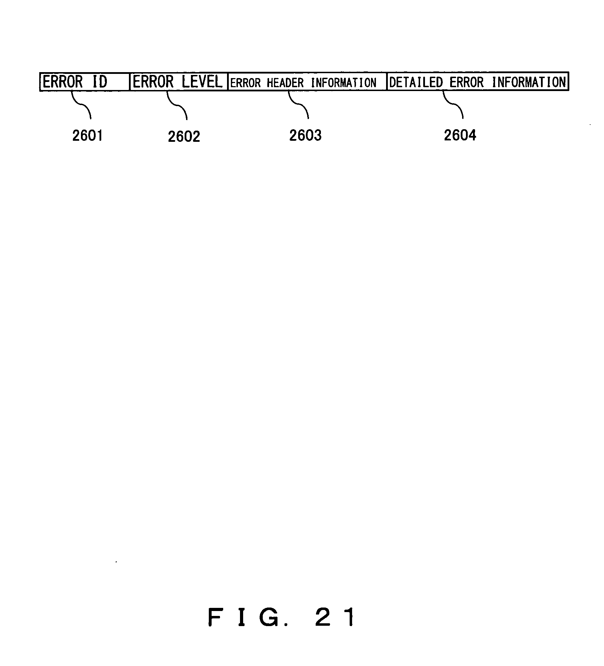

Fig. 21 shows an example of a data structure of the error information Eg according to the present embodiment. The error information Eg includes an "error ID" 2601, an "error level" 2602, "error header information" 2603, and "error detailed information" 2604 . The "error ID" 2601 is an ID used for identifying the error information Eg. The "error level" 2602 is an error level according to the importance of each error (level S, level A, and level B). The "error header information" 2603 is simplified information about each error, and is displayed on the error display area 2501. The "error detailed information" 2604 is detailed information about each error.

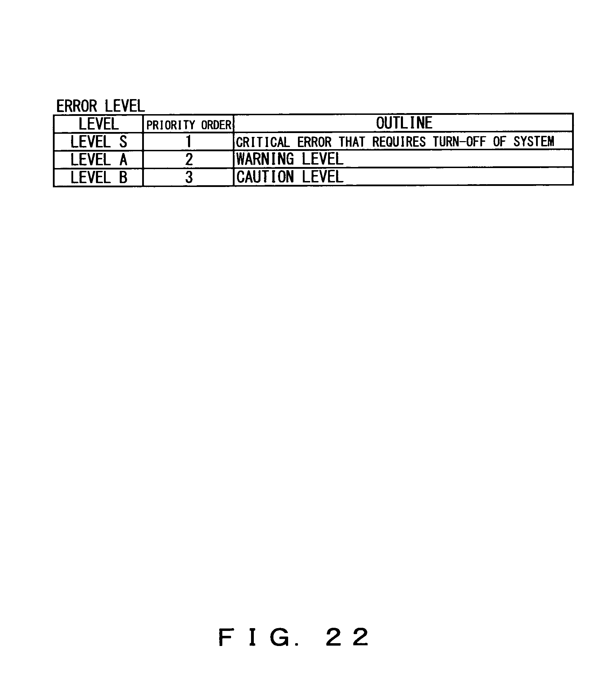

Fig. 22 shows the respective error levels according to the present embodiment. There are level S, level A, and level B, starting from the level of the highest priority. Level S represents a critical error that requires a turn-off of the system. Level A represents a warning. Level B represents caution.

The priority order is the order of displaying the pieces of information. If an error E_{H} is caused when other error information E_{R} of a lower priority order is being displayed (case 1), the error information E_{H} of the higher priority order is displayed. If error information E_{S} is caused when error information E_{R} of the same priority order is being displayed (case 2), E_{S} and E_{R} are displayed sequentially and repeatedly. If error E_{L} is caused when an error E_{R} of a higher priority order is being displayed (case 3), the error E_{L} of the lower priority order is not displayed unless the error E_{R} is cancelled.

When a level S error is caused, only the error information of that error is continuously displayed on the error display area 2501, and all the operations and all the communications with other devices are stopped. Also, even if a prescribed button (for example, a "Help" button or the like) is not pressed, the content of the "error detailed information" 2604 is displayed on the central portion of the TP 221 and the display panel window.

If a level A error is caused when a level B error is being displayed, the level A error information is displayed on the error display area 2501. The level B error is not displayed on the error display area 2501 until the level A error is cancelled.

If another level A error is caused when a level A error is being displayed, the latest level A error is displayed on the error display area 2501 for "n" seconds (n is an integer set arbitrarily) . Thereafter, the other pieces of level A error information are displayed sequentially. This is also applied to a case when a level B error is newly caused when a level B error is being displayed.

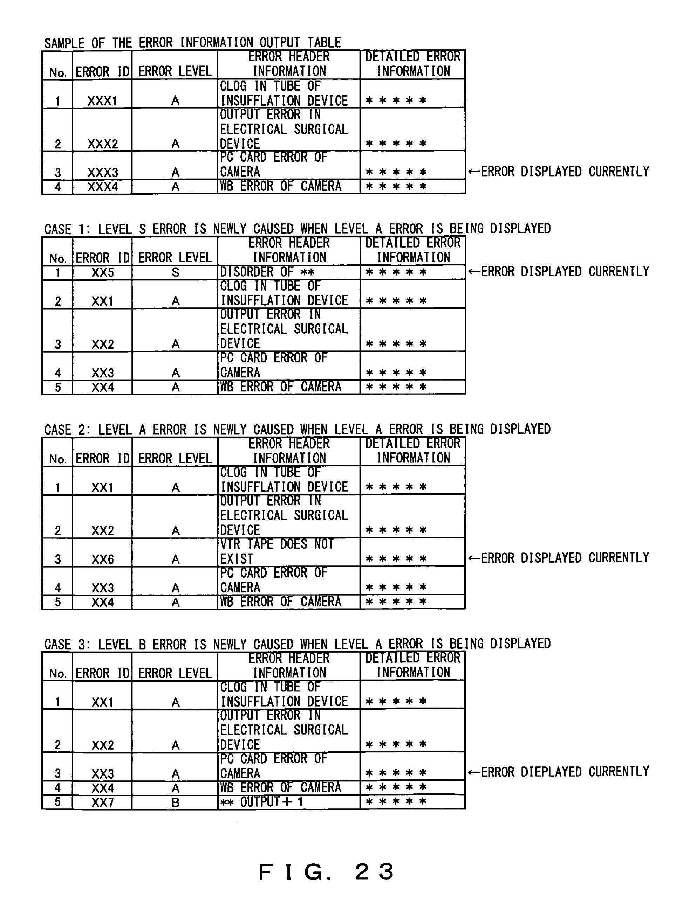

Fig. 23 shows how the registered positions change according to error levels when registration is made on an error information output table Tb1. The error information output table Tb1 is stored in a prescribed storage device in the TP control switching unit 305. The first table in Fig. 23 is a sample of the error information output table Tb1. On this error information output table Tb1, four pieces of level A error information are registered as records No. 1 through No. 4. The error information of record No. 3 is currently displayed. Thereafter, the records are sequentially and repeatedly displayed in the order of No. 4 → No. 1 → No . 2 → No. 3 → No. 4 → No. 1 →....... The respective pieces of error information are displayed for "n" seconds. Hereinafter, cases 1-3 are explained by using this sample.

First explanation is given for a case in which an error E_{H} is caused when error information E_{R} of a lower priority order is being displayed (case 1) . If a level S error is caused (error ID XX5) when a level A error is being displayed, the record of the level S error information is inserted into the top of the error information output table Tb1, and the records below the inserted position are respectively shifted to the lower positions. Then, the level S error information is output from the TP control switching unit 305 to the TP 221.

Next, explanation is given for a case in which an error E_{S} is caused when error information E_{R} of the same priority order is being displayed (case 2). If a level A error (error ID: XX5, VTR tape does not exist) is caused when level A error information of the same priority order (camera PC card error) is being displayed, the record of the error information of "error ID: XX6" is inserted into the position of record No. 3, and the records below that position are respectively shifted to the lower positions. Then, the error information of the record No. 3 that was newly inserted is displayed for n seconds. Thereafter, the records are sequentially and repeatedly displayed in the order of No. 4 → No. 5 → No. 1 → No. 2 → No. 3 → No. 4 → No. 5 → No. 1.......

Explanation is given for a case in which an error E_{L} is caused when error information E_{R} of a higher priority order is being displayed (case 3). If a level B error (error ID: XX7) is caused when a level A error is being displayed, the record of the level B error information is added to the bottom of the error information output table Tb1. In this case, the records are sequentially and repeatedly displayed in the order of No. 3 → No. 4 → No. 1 → No. 2 → No. 3 → No. 4 → No. 1....... The level B error (error ID: XX7) is not displayed unless all the pieces of the level A error information are cancelled.

When errors are cancelled, the records of the pieces of error information corresponding to the cancelled errors are deleted from the error information output table Tb1, and the records that were below the deleted positions are respectively shifted to the upper positions in order to fill the deleted positions. When an error that is currently being displayed is deleted, the next error is displayed immediately.

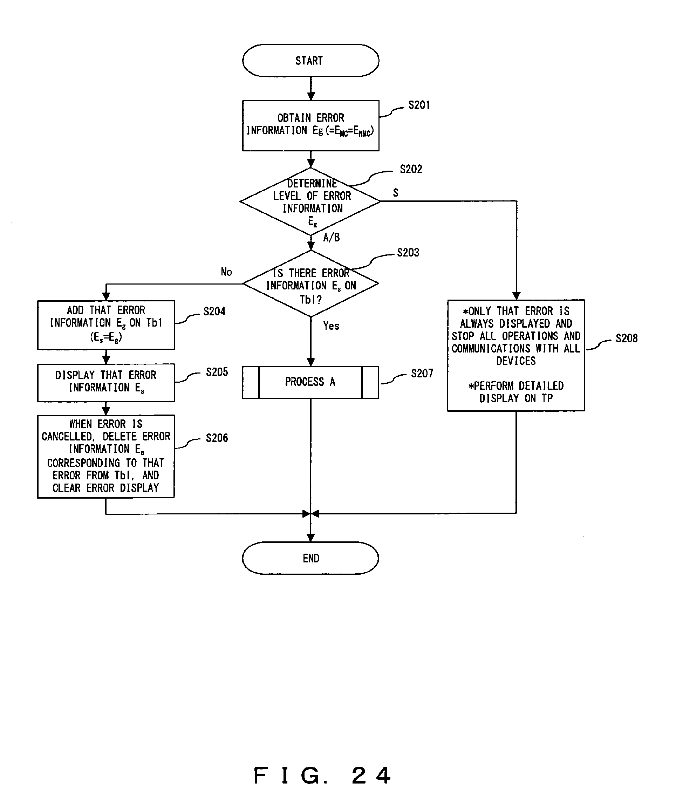

Fig. 24 is a flowchart for outputting error information according to the present embodiment. The programs for the present embodiment are respectively stored in the storage devices in the MC 114 and the storage device 306 in the NMC 202. The control unit 300 and the control unit 400 respectively read the programs from their own storage devices, and execute the flowchart shown in Fig. 24. For simplicity of explanation, constituent elements of the controller that is selected as the control target by the TP 221 are represented by a control unit Ca, an error detection unit C1a, an error display process unit C2a, and a GUI creation unit C3a, and constituent elements of the other controller are represented by a control unit Cb, an error detection unit C1b, an error display process unit C2b, and a GUI creation unit C3b.

Accordingly, in the case shown in Fig. 19A, the control unit 400, the error detection unit 403, the error display process unit 402, and the GUI creation unit 401 respectively correspond to Ca, C1a, C2a, and C3a. Also, the control unit 300, the error detection unit 323, the error display process unit 322, and the GUI creation unit 321 respectively correspond to Cb, C1b, C2b, and C3b.

Also, in the case shown in Fig. 19B, the control unit 300, the error detection unit 323, the error display process unit 322, and the GUI creation unit 321 respectively correspond to Ca, C1a, C2a, and C3a. Also, the control unit 400, the error detection unit 403, the error display process unit 402, and the GUI creation unit 401 respectively correspond to Cb, C1b, C2b, and C3b.

First, the error display process unit C2a obtains the error information E_{MC} and E_{NMC} detected in the error detection units C1a and C1b (S201). The error display process unit C2a determines the error levels of the obtained error information Eg (= E_{MC} = E_{NMC}) (S202).

When it is determined in S202 that the error level is level S, the error display process unit C2a causes only that error to be displayed, and stops all the operations and all the communications with other devices. Further, details of that error are displayed on the TP 221 (S208).

When it is determined in S202 that the error level is level A/B, the error display process unit C2a determines whether there is error information E_{S} that is registered currently in the error information output table Tb1 (S203).

When there is no error information Eₑ that is registered currently on the error information output table Tb1 (No in S203), the error display process unit C2a adds the obtained error information Eg to the error information output table Tb1 (S204), and outputs it to the TP 221 (S205). When the error is cancelled, the error display process unit C2a deletes from Tb1 the error information Eₑ corresponding to the cancelled error, and clears the display of the error on the TP 221 (S206).

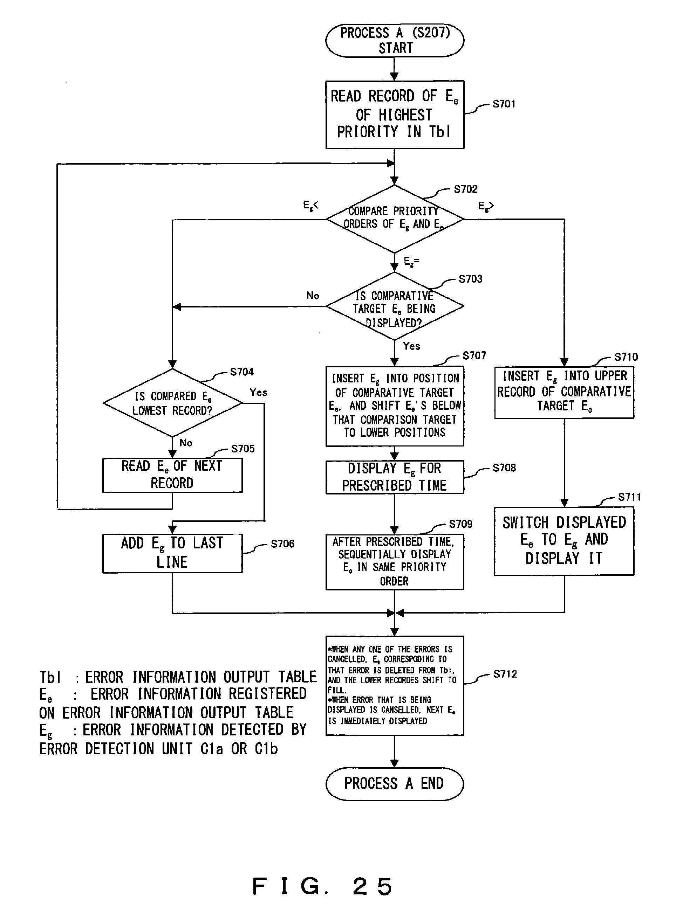

When there is error information Es currently registered on the error information output table Tb1 (Yes in S203), the error display process unit C2a performs process A (S207). Process A is explained in Fig. 25.

Fig. 25 shows, in detail, process A (S207) shown in Fig. 24. The error display process unit C2a reads the error information Es of the top record in the error information output table Tb1 (S701), and executes the processes described below.

First, the priority order of the obtained error information Eg and the priority order of the error information Eₑ read from the error information output table Tb1 are compared to each other (S702). When Eg < Eₑ is satisfied in S702, the record of the error information Eg is added (S706) to the record next to the error information Eₑ on the condition that the error information Eₑ that was compared with was the lowest record (Yes in S704). If the error information Eₑ that was compared with was not the lowest record (No in S704), the error information Eₑ of the next record is read (S705), and the process of S702 is executed.

When Eg = Eₑ is satisfied in S702, the process proceeds to S704 on the condition that the error information Eₑ as a comparative target is not displayed on the TP 221 (No in S703). When the comparative target Eₑ is displayed on the TP 221 (Yes in S703), the error information Eg is inserted into the record position of the comparative target Eₑ, and the records below the inserted record are respectively shifted to the lower positions (S707).

The inserted error information Eg is displayed for a prescribed time on the TP 221 (S708) . Afteraprescribed time has elapsed, pieces of error information Eₑ that are of the same priority order are respectively displayed for a prescribed time on the TP 221 (S709).

When Eg > Eₑ is satisfied in S702, the record of the error information Eg is inserted into the position before the record of the comparative target Eₑ (S710). Then, the error information Eₑ being currently displayed on the TP 221 is switched to the error information Eg to be displayed (S711).

When any one of the errors is cancelled, the record of the error information Eₑ corresponding to the cancelled error is deleted, and the records below the record of the cancelled error are respectively shifted to the upper positions. Also, when the error that is currently displayed is cancelled, the next error information Eₑ is immediately displayed (S712).

As described above, the medical support control system according to the present embodiment includes the first controller (MC 114) connected to at least one device, the second controller (NMC 202) connected to at least one device, and the manipulation display device (TP 221).

The manipulation device (TP 221) is shared by the first controller and the second controller. The manipulation display device (TP 221) can alternately display the first graphical user interface (first GUI) created by the first controller and the second graphical user interface (second GUI) created by the second controller.

In the medical support control system, the first and second controllers share the first error information (E_{MC}), which is error information obtained from the first controller side, and the second error information (E_{NMC}), which is error information obtained from the second controller side. Further, the medical support control system can reflect the first and second error information on the first and second GUIs on the basis of the content of the first and second error information. The first and second information has priority order information and error content information representing the content of an error.

The error content information of the error information of the highest priority order among the obtained error information is displayed on the manipulation display device. If the priority order of the obtained pieces of error information are the same, the pieces of error content information of the error information of the same priority orders are sequentially switched to be displayed on the manipulation devices.

According to the present embodiment, not only can errors caused in the controller that is the control target of the TP 221 be displayed, but errors caused in the controller that is not the control target of the TP 221 can also be displayed.

The scope of the present invention is not limited to any of the above embodiments, and various other configurations and embodiments are allowed without departing from the spirit of the present invention.

As described above, it is possible to provide a medical support control device for controlling medical devices and non-medical devices.

## Claims

1. A medical support control system, comprising:
a first controller (114) connected to at least one device;
a second controller (202) connected to at least one device; and
a manipulation display device (221) shared by the first and second controllers, wherein:
the manipulation display device (221) is caused to alternately display a first graphical user interface created by the first controller (114) and second graphical user interface created by the second controller (202).

2. The medical support control system according to claim 1, wherein:
the first controller (114) has first identification information and user environment information corresponding to the first identification information;
the second controller (202) has second identification information and user environment information corresponding to the second identification information; and
when log-in to at least one of the first and second controllers has succeeded, an operation environment for the controller to which the log-in has succeeded is set in accordance with the user environment information.

3. The medical support control system according to claim 2, wherein:
an operation environment is set for controllers other than the controller to which the log-in has succeeded, in accordance with user environment information common to all users.

4. The medical support control system according to claim 1, wherein:
the identification information includes at least an operating person name and a procedure name; and
the user environment information is operation environment information of the controller to which the log-in has succeeded, used for setting an operation environment appropriate for a procedure performed by an operating person identified by the operating person name and the procedure name.

5. A medical support control system, comprising:
a first controller (114) connected to at least one device;
a second controller (202) connected to at least one device, connected to a network, and provided in a medical environment different from that of the first controller; and
a manipulation display device (221) shared by the first and second controllers and alternately displaying a first graphical user interface (first GUI) created by the first controller and a second graphical user interface (second GUI) created by the second controller,
wherein:
if a prescribed report is received via the network or from the prescribed connected device while the first GUI is being displayed on the manipulation display device, a symbol (1503, 1702, 1802) indicating that the report has been received is displayed on the first GUI, and when the symbol is pressed, the first GUI is switched to a prescribed window of the second GUI corresponding to the report, and a process corresponding to the report is performed by the second controller.

6. The medical support control system according to claim 5, wherein:
the network is a telephone circuit (1350); and
when there is a telephone call via the telephone circuit while the first GUI is being displayed on the manipulation display device (221), a symbol (1503) indicating that there is a telephone call is displayed on the first GUI, and when the symbol (1503) is pressed, the first GUI is switched to a conversation window (1601) of the second GUI, and a conversation via the second controller is enabled.

7. The medical support control system according to claim 5, wherein:
when an e-mail has been received via the network while the first GUI is being displayed on the manipulation display device (221), a symbol (1702) indicating that the e-mail has been received is displayed on the first GUI, and when the symbol (1702) is pressed, the first GUI is switched to an e-mail transmission/reception window (1703) of the second GUI, and the received e-mail is displayed in the e-mail transmission/reception window (1703) by the second controller.

8. The medical support control system according to claim 5, wherein:
the prescribed device is a video recorder (1800); and
when a report indicating that there is no more free space in a storage medium is received from the video recorder (1800) while the first GUI is being displayed on the manipulation display device (221), a symbol (1802) indicating reception of the report is displayed on the first GUI, and when the symbol (1802) is pressed, the first GUI is switched to a video recorder manipulation window (1803) of the second GUI, and the second controller causes the video recorder to perform operations for replacing the storage medium with a new one.

9. The medical support control system according to claim 5, wherein:
the second controller (202) has a switching unit for (305) switching a GUI to be displayed on the manipulation display device (221) between the first GUI and the second GUI; and
the switching unit (305) switches a GUI to be displayed on the manipulation display device (221) from the first GUI to the second GUI in accordance with a report from the first controller indicating that the symbol was pressed.
